# EUROPEAN PATENT APPLICATION

(11) **EP 4 450 507 A1**
(43) Date of publication of application: **23.10.2024**
(21) Application number: 23752637.1
(22) Date of filing: 20.01.2023
(51) Int. Cl.: C07D 498/22, C07D 209/86, C07D 213/16, C07D 251/24, C07D 307/91, C07D 311/86, C07D 333/76, C07D 335/16, C07D 403/10, C07D 407/14, C07D 409/14, C07D 487/14, C07D 487/22, C07D 513/22, C07D 517/22, C09K 11/06, G09F 9/30, H05B 33/02, H10K 50/00, H10K 59/00

(54) **ORGANIC COMPOUND AND ORGANIC LIGHT-EMITTING ELEMENT**

(30) Priority: 08.02.2022 JP 2022018104; 14.12.2022 JP 2022199794
(71) Applicant: CANON KABUSHIKI KAISHA, Tokyo 146-8501 (JP)
(72) Inventor: NISHIDE Yosuke, Tokyo 146-8501 (JP); KAMATANI Jun, Tokyo 146-8501 (JP); YAMADA Naoki, Tokyo 146-8501 (JP); IWAWAKI Hironobu, Tokyo 146-8501 (JP); OHRUI Hiroki, Tokyo 146-8501 (JP); HORIUCHI Takayuki, Tokyo 146-8501 (JP); MIYASHITA Hirokazu, Tokyo 146-8501 (JP)
(74) Representative: WESER & Kollegen Patentanwälte PartmbB
(86) International application number: PCT/JP2023/001648
(87) International publication number: WO 2023/153169

(57) **Abstract**

The present disclosure provides an organic compound represented by general formula(1): 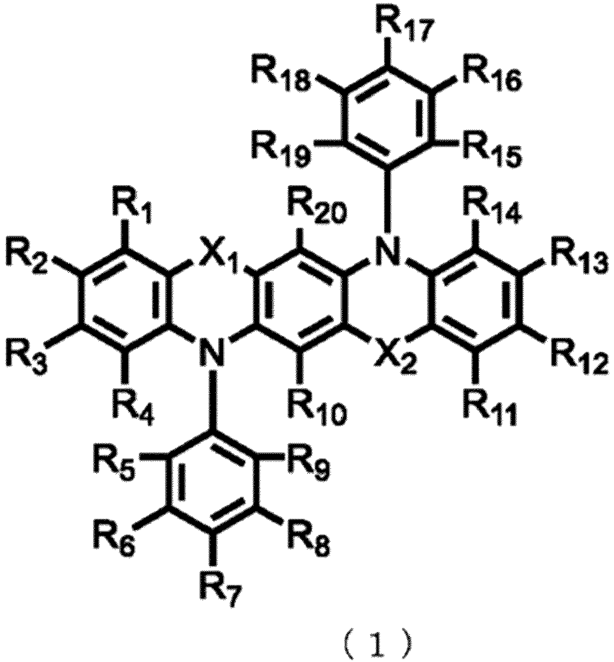 (In general formula (1), R₁ to R₂₀ are each independently selected from the group consisting of a hydrogen atom, a deuterium atom, halogen atoms, alkyl groups, alkoxy groups, silyl groups, amino groups, aryl groups, heteroaryl groups, and a cyano group. R₁ to R₂₀ may be bonded to one another to form a ring and, optionally, via a chalcogen atom. At least a combination R₉ and R₁₀ or R₁₉ and R₂₀ forms a bond. X₁ and X₂ are each independently selected from the group consisting of chalcogen atoms, imino groups, methylene groups, and silylene groups.)

## Description

### Technical Field

The present invention relates to an organic compound and an organic light-emitting element using the same.

### Background Art

An organic light-emitting element (hereinafter also referred to as "organic electroluminescent element" or "organic EL element") includes a pair of electrodes and an organic compound layer disposed between the electrodes. Electrons and holes are injected into the organic compound layer from the electrodes, thereby generating excitons of the luminescent organic compound in the organic compound layer. When the excitons return to the ground state, the organic light-emitting element emits light.

Organic light-emitting elements have recently advanced remarkably and feature low driving voltage, a variety of emission wavelengths, fast response, and the possibility of achieving thinner and lighter-weight light-emitting devices.

In addition, color gamuts reproduced by displays have been specified by sRGB or Adobe RGB standards and require materials that reproduce the colors of these standards, and BT-2020 has recently been used as a standard that further expands the color gamut.

Luminescent organic compounds have been actively developed up to now.
Developing highly luminescent compounds is important for providing a high-performance organic light-emitting element.

The following compound 1-a, which is disclosed in PTL 1, is a compound that has been developed so far.

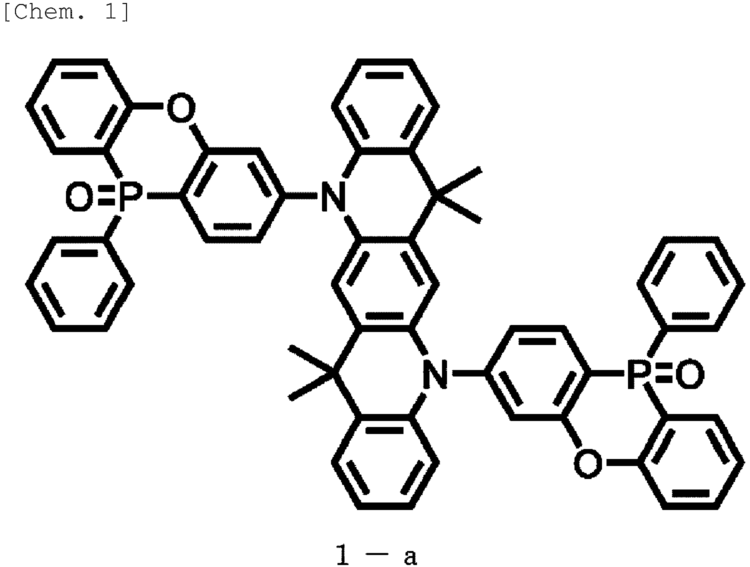

### Citation List

### Patent Literature

PTL 1: U.S. Patent Application Publication No. 2017/0256717
PTL 2: Chinese Patent Application Publication No. 107043382

### Summary of Invention

### Technical Problem

PTL 1 discloses Compound 1-a as a luminescent material, but Compound 1-a has an issue with color purity.

Accordingly, the present invention provides an organic compound with excellent color purity.

### Solution to Problem

The organic compound of the present invention is represented by general formula (1):

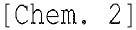

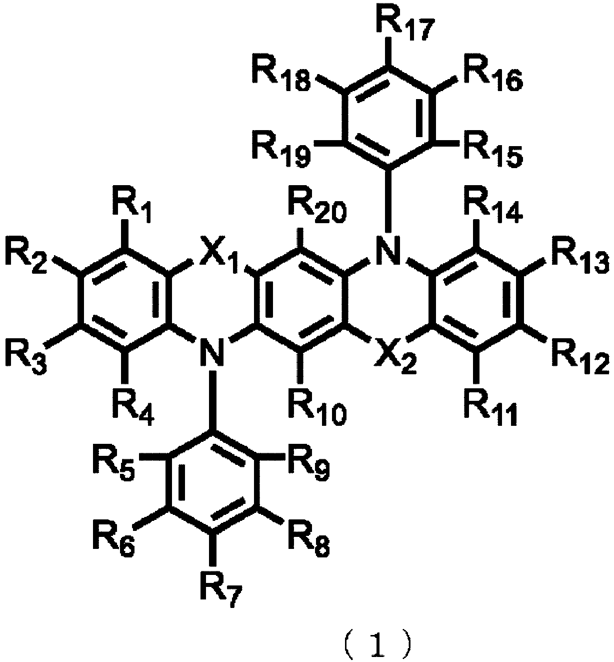

In general formula (1), R₁ to R₂₀ are each independently selected from the group consisting of a hydrogen atom, a deuterium atom, halogen atoms, substituted or unsubstituted alkyl groups, substituted or unsubstituted alkoxy groups, substituted or unsubstituted silyl groups, substituted or unsubstituted amino groups, substituted or unsubstituted aryl groups, substituted or unsubstituted heteroaryl groups, and a cyano group.

R₁ to R₂₀ may be bound to one another to form a ring and, optionally, via a chalcogen atom. At least a combination R₉ and R₁₀ or R₁₉ and R₂₀ forms a bond.

X₁ and X₂ are each independently selected from the group consisting of chalcogen atoms, substituted or unsubstituted imino groups, substituted or unsubstituted methylene groups, and substituted or unsubstituted silylene groups. Advantageous Effects of Invention

The organic compound of the present invention has a fused ring structure, which enables the organic compound to emit blue light with excellent color purity.

### Brief Description of Drawings

[Fig. 1A] Fig. 1A is a schematic sectional view of a pixel of a display device according to an embodiment of the present invention.
[Fig. 1B] Fig. 1B is a schematic sectional view of a display device using an organic light-emitting element according to an embodiment of the present invention.
[Fig. 2] Fig. 2 is a schematic illustrative representation of a display device according to an embodiment of the present invention.
[Fig. 3A] Fig. 3A is a schematic illustrative representation of an imaging device according to an embodiment of the present invention.
[Fig. 3B] Fig. 3B is a schematic illustrative representation of an electronic apparatus according to an embodiment of the present invention.
[Fig. 4A] Fig. 4A is a schematic illustrative representation of a display device according to an embodiment of the present invention.
[Fig. 4B] Fig. 4B is a schematic illustrative representation of an example of foldable display devices.
[Fig. 5A] Fig. 5A is a schematic illustrative representation of a lighting device according to an embodiment of the present invention.
[Fig. 5B] Fig. 5B is a schematic illustrative representation of an automobile including a lighting fixture for vehicles according to an embodiment of the present invention.
[Fig. 6A] Fig. 6A is a schematic illustrative representation of a wearable device according to an embodiment of the present invention.
[Fig. 6B] Fig. 6B is a schematic illustrative representation of a wearable device according to an embodiment of the present invention, including an imaging device.
[Fig. 7A] Fig. 7A is a schematic illustrative representation of an image forming apparatus according to an embodiment of the present invention.
[Fig. 7B] Fig. 7B is a schematic illustrative representation of an example of the exposure light source of the image forming apparatus according to an embodiment of the present invention.
[Fig. 7C] Fig. 7C is a schematic illustrative representation of another example of the exposure light source of the image forming device according to an embodiment of the present invention.

### Description of Embodiments

In the description herein, halogen atoms include, but are limited to, fluorine, chlorine, bromine, iodine, astatine, and tennessine.

Chalcogen atoms include, but are not limited to, oxygen, sulfur, selenium, tellurium, polonium, and livermorium.

The alkyl group may have 1 to 20 carbon atoms, and examples include, but are not limited to, methyl, ethyl, n-propyl, isopropyl, n-butyl, tert-butyl, sec-butyl, octyl, cyclohexyl, tert-pentyl, 3-methylpentan-3-yl, 1-adamantyl, and 2-adamantyl.

The alkoxy group may have 1 to 10 carbon atoms, and examples include, but are not limited to, methoxy, ethoxy, propoxy, isopropoxy, tert-butoxy, 2-ethyl-octyloxy, and benzyloxy.

The aryl group may have 6 to 20 carbon atoms, and examples include, but are not limited to, phenyl, naphthyl, indenyl, biphenyl, terphenyl, fluorenyl, phenanthryl, triphenylenyl, pyrenyl, anthranil, perylenyl, chrysenyl, and fluoranthenyl.

The heteroaryl group may have 3 to 20 carbon atoms, and examples include, but are not limited to, pyridyl, pyrimidyl, pyrazyl, triazyl, benzofuranyl, benzothiophenyl, dibenzofuranyl, dibenzothiophenyl, oxazolyl, oxadiazolyl, thiazolyl, thiadiazolyl, carbazolyl, acridinyl, and phenanthrolyl.

Silyl groups include, but are not limited to, trimethylsilyl and triphenylsilyl.

Examples of the amino group include, but are not limited to, N-methylamino, N-ethylamino, N,N-dimethylamino, N,N-diethylamino, N-methyl-N-ethylamino, N-benzylamino, N-methyl-N-benzylamino, N,N-dibenzylamino, anilino, N,N-diphenylamino, N,N-dinaphthylamino, N,N-difluorenylamino, N-phenyl-N-tolylamino, N,N-ditolylamino, N-methyl-N-phenylamino, N,N-dianisolylamino, N-mesityl-N-phenylamino, N,N-dimesitylamino, N-phenyl-N-(4-tert-butylphenyl)amino, N-phenyl-N-(4-trifluoromethylphenyl)amino, N-piperidyl, carbazolyl, acridyl, trimethylamino, and triphenylamino.

Examples of the substituents that the above-mentioned alkyl, alkoxy, amino, aryl, heterocyclic, aryloxy, and silyl groups may further have include, but are not limited to, deuterium; alkyl groups, such as methyl, ethyl, n-propyl, isopropyl, n-butyl, and tert-butyl; aralkyl groups, such as benzyl; aryl groups, such as phenyl and biphenyl; heterocyclic groups, such as pyridyl and pyrrolyl; amino groups, such as dimethylamino, diethylamino, dibenzylamino, diphenylamino, and ditolylamino; alkoxy groups, such as methoxy, ethoxy, and propoxy; aryloxy groups, such as phenoxy; halogen atoms, such as fluorine, chlorine, bromine, and iodine; and a cyano group.

### (1) Organic Compound

The organic compound of the present invention will first be described.

The organic compound of the present invention is represented by general formula (1): In the description provided herein, the main skeleton refers to a skeleton that includes a structure formed by fusing three benzene rings and two six-membered rings containing chalcogen atoms. Also, the basic skeleton refers to a skeleton having a structure in which at least one of the substituents at the two nitrogen atoms is fused to the main skeleton.

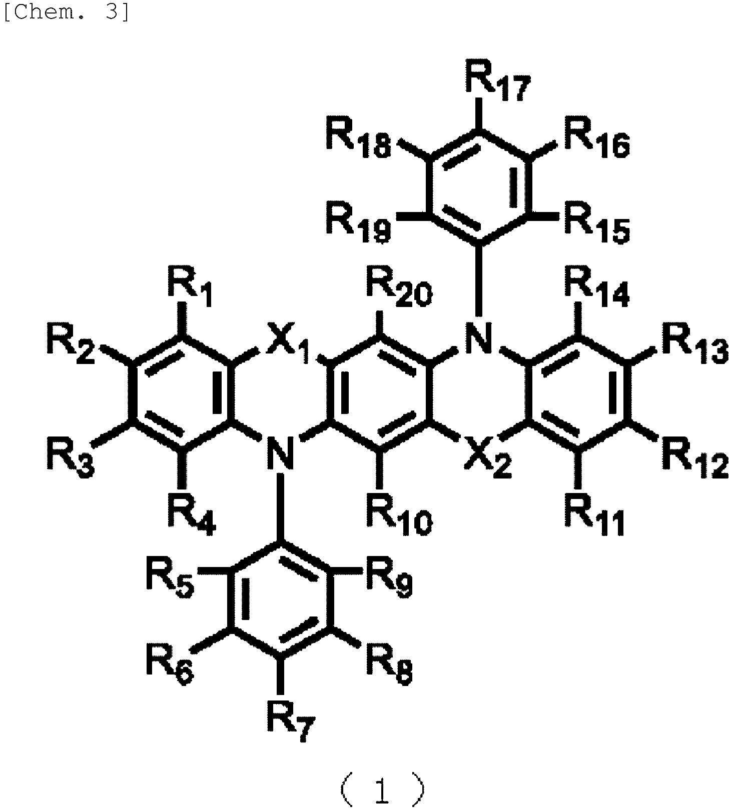

In general formula (1), R₁ to R₂₀ are each independently selected from the group consisting of a hydrogen atom, a deuterium atom, halogen atoms, substituted or unsubstituted alkyl groups, substituted or unsubstituted alkoxy groups, substituted or unsubstituted silyl groups, substituted or unsubstituted amino groups, substituted or unsubstituted aryl groups, substituted or unsubstituted heteroaryl groups, and a cyano group.

R₁ to R₂₀ may be bound to one another to form a ring and, optionally, via a chalcogen atom. The chalcogen atom intervening in such binding is preferably oxygen, sulfur, or selenium, more preferably oxygen or sulfur. At least a combination R₉ and R₁₀ or R₁₉ and R₂₀ forms a bond.

X₁ and X₂ are each independently selected from the group consisting of chalcogen atoms, substituted or unsubstituted imino groups, substituted or unsubstituted methylene groups, and substituted or unsubstituted silylene groups. The chalcogen atoms as X₁ and X₂ are preferably oxygen, sulfur, or selenium and particularly preferably oxygen or sulfur.

Preferably, the organic compound of the present invention has the structure represented by general formula (2) or (3).

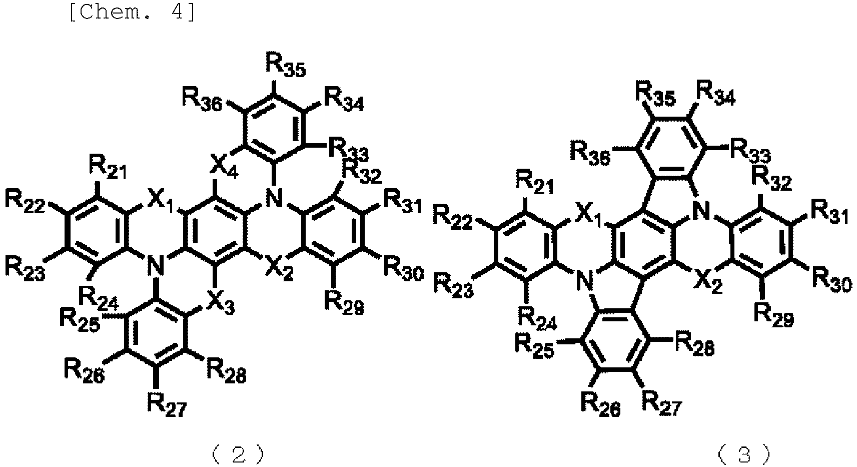

In general formulas (2) and (3), R₂₁ to R₃₆ are each independently selected from the group consisting of a hydrogen atom, a deuterium atom, halogen atoms, substituted or unsubstituted alkyl groups, substituted or unsubstituted alkoxy groups, substituted or unsubstituted silyl groups, substituted or unsubstituted amino groups, substituted or unsubstituted aryl groups, substituted or unsubstituted heteroaryl groups, and a cyano group.

R₂₁ to R₃₆ may be bonded to one another to form a ring and, optionally, via a chalcogen atom. The chalcogen atom intervening such binding is preferably oxygen, sulfur, selenium, or tellurium, more preferably oxygen or sulfur, and particularly preferably oxygen.

X₁ to X₄ are each independently selected from the group consisting of chalcogen atoms, substituted or unsubstituted imino groups, substituted or unsubstituted methylene groups, and substituted or unsubstituted silylene groups. Preferably, X₁ to X₄ are oxygen, sulfur, selenium, or tellurium atoms, more preferably oxygen or sulfur atoms, and particularly preferably oxygen atoms.

Particularly preferably, the organic compound of the present invention has the structure represented by general formula (3).

In an embodiment of the present invention, the organic compound may have no structure represented by general formula (4).

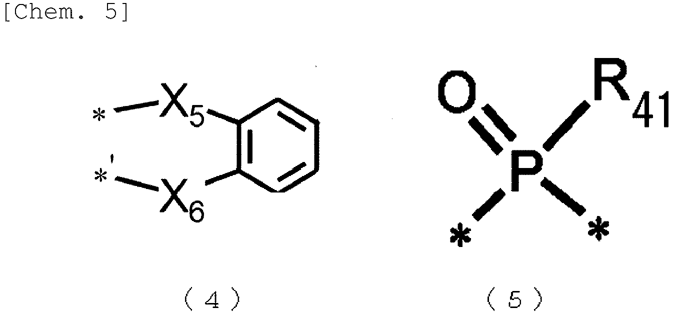

In general formula (4), X₅ and X₆ are selected from an oxygen atom, a sulfur atom, a dimethylmethylene group, a sulfonyl group, or the structure represented by general formula (5). The asterisk * indicates the position of a bond to R₂₆ or R₃₄, and *' indicates the position of a bond to R₂₇ or R₃₅.

In general formula (5), R₄₁ represents a phenyl group. The asterisks * indicate binding positions.

The organic compounds represented by general formulas (1) to (3) have the following features:
(1-1) The basic skeleton has a structure in which at least one of the substituents at the two nitrogen atoms in the main skeleton is fused to the main skeleton, and this structure enables blue emission with excellent color purity.
(1-2) The basic skeleton is formed of a plurality of fused ring structures, leading to high thermal stability.

The characteristic features of the basic skeleton of the organic compound of the present invention will now be described in comparison with a comparative compound having a similar structure to the organic compound according to the present invention. Specifically, comparative compound 1-a and exemplified compounds of embodiments of the present invention are cited.

(1-1) The basic skeleton has a structure in which at least one of the substituents at the two nitrogen atoms in the main skeleton is fused to the main skeleton, and this structure enables blue emission with excellent color purity.

At the beginning of inventing the organic compound represented by general formula (1), the present inventors focused on the basic skeleton itself.

First, in order to emit blue light with good color purity, the basic skeleton itself is required to produce emission in the highly pure blue region. In the embodiments of the present invention, blue emission with excellent color purity as desired means that the organic compound in a dilute toluene solution exhibits an emission spectrum with a maximum emission wavelength of 440 nm to 475 nm and a half-width of 50 nm or less. Furthermore, a half-width of 40 nm or less is preferred for blue emission with more excellent color purity. The half-width of an emission spectrum, used herein, refers to the width in the spectrum at an intensity of 0.5 when the maximum intensity is defined as 1.0.

The exemplified compounds according to the present invention have structures in which at least one of the substituents at the two nitrogen atoms is fused to the main skeleton. The inventors found that this structure reduces molecular vibrations and structural changes between the excited and ground states compared to the structures in which the substituent is not fused to the main skeleton, consequently narrowing the half-width in the emission spectrum.

In contrast, comparative compound 1-a has C-N bonds with a rotation axis at two positions, and the substituents at the two nitrogen atoms in the main skeleton are both not fused to the main skeleton. Accordingly, vibrations caused by the C-N bonds and structural changes between the excited and ground states affect the emission spectrum and expand the half-width.

Table 1 presents comparisons among emission spectra of exemplified compounds according to the present invention and the comparative compound in dilute toluene solutions. More specifically, after measuring emission spectra, the half-widths of the obtained emission spectra and emission colors were compared. The emission wavelength was determined by measuring the photoluminescence of the dilute toluene solution at an excitation wavelength of 350 nm at room temperature using F-4500 manufactured by Hitachi. For the emission spectrum half-widths in Table 1, A is assigned for widths of 40 nm or less, B is assigned for widths of 50 nm or less, and C is assigned for widths of larger than 50 nm. AA indicates more excellent color purity than A.

### [Table 1]

**Table 1**

| | Structural formula | Emission spectrum half-width | Emission color |
|---|---|---|---|
| Exemplified Compound A2 | 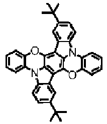 | AA | Blue |
| Exemplified Compound A11 | 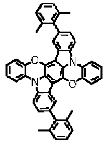 | AA | Blue |
| Exemplified Compound B2 | 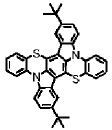 | A | Blue |
| Exemplified Compound C2 | 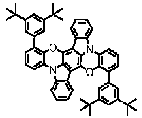 | A | Blue |
| Exemplified Compound C12 | 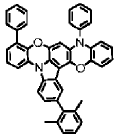 | B | Blue |
| Comparative Compound 1-a | 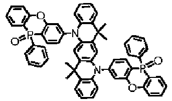 | C | Light blue |

Table 1 shows that in contrast to Comparative Compound 1-a, the exemplified compounds according to the present invention have structures in which at least one of the substituents at the two nitrogen atoms is fused to the main skeleton, suggesting that such a structure has a half-width of 50 nm or less, which is required for blue emission with excellent color purity. Thus, the compound according to the present invention emits blue light with excellent color purity as a unique effect of the structure in which at least one of the substituents at the two nitrogen atoms in a main chain is fused to the main chain. In particular, it is preferable that both the substituents at the two nitrogen atoms in the main chain are fused.

### (1-2) The basic skeleton is formed of a plurality of fused ring structures, leading to high thermal stability.

The compounds in the organic layers, particularly in the luminescent layer, of an organic light-emitting element repeat the transition between the ground and excited states, particularly in the luminescent layer, while the organic light-emitting element emits light. In this process, molecules stretch, contract, rotate, and so forth. If the molecules have a site where the bond is easily dissociated, the bond may be cleaved, and a portion of the compound is liberated. Such a partial liberation of a compound changes the structure, and hence, compounds that are prone to cause liberation are less durable. If such a compound is used in an organic light-emitting element, the liberated portion acts as a quencher and thus degrades the durability of the element. Thus, molecules having a structure in which bonds are less likely to be dissociated and less likely to cause liberation provide more durable elements.

Table 2 shows differences in structure between Exemplified Compound A2, according to the present invention, and Comparative Compound 1-a. Exemplified Compound A2, according to the present invention, has the basic skeleton, hence having C-C bonds with high bonding energy in addition to the C-N bonds with low bonding energy. On the other hand, Comparative Compound 1-a merely has C-N bonds with low bonding energy. Thus, the organic compound of the present invention has higher binding stability than Comparative Compound 1-a and, accordingly, can improve the durability of the element.

### [Table 2]

**Table 2**

| | Structural formula | Type of bond Bonding energy |
|---|---|---|
| Exemplified Compound A2 | 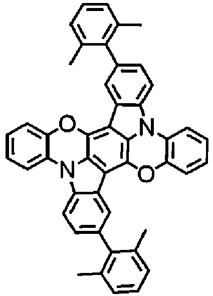 | C-C 5.0eV |
| Comparative Compound 1-a | 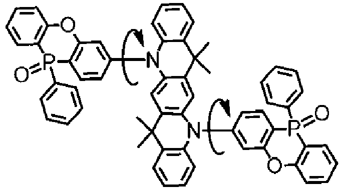 | C-N 3.9eV |

Preferably, the organic compound according to the present invention further has the following features:
(1-3) At least one of R₂₁, R₂₂, R₂₃, R₂₆, R₂₇, R₂₉, R₃₀, R₃₁, R₃₄, and R₃₅ of general formulas (2) and (3) is other than the hydrogen atom, that is, a substituent, thereby improving durability; and
(1-4) At least either R₂₁ or R₂₉ of general formulas (2) and (3) is a substituent, thereby reducing concentration quenching.

These features will now be described.

(1-3) At least one of R₂₁, R₂₂, R₂₃, R₂₆, R₂₇, R₂₉, R₃₀, R₃₁, R₃₄, and R₃₅ of general formulas (2) and (3) is other than the hydrogen atom, that is, a substituent, thereby improving durability.

Table 3 presents the calculation results of the basic skeleton twist angles and S1 (lowest excited singlet energy) when a phenyl group is introduced to each substitution position of general formula (3). The basic skeletal twist angle is an indicator of the planarity of the basic skeleton. The smaller the basic skeletal twist angle, the higher the planarity. Table 3 shows that the basic skeletons in which at least one of the substitution positions R₂₁, R₂₂, R₂₃, R₂₆, and R₂₇ is a substituent exhibit small twist angles and are thus favorable, suggesting that it is preferable that at least one of R₂₂, R₂₃, R₂₆, and R₂₇, particularly at least one of R₂₂, R₂₃, and R₂₇, is a substituent. In particular, when at least one of the substitution positions R₂₂, R₂₃, and R₂₇ is a substituent, S1 is high. A small basic skeleton twist angle leads to a reduced half-width in the emission spectrum, suggesting excellent durability. Also, a large S1 value suggests blue emission with high color purity. Thus, in general formulas (2) and (3), it is preferable that at least one of R₂₁, R₂₂, R₂₃, R₂₆, R₂₇, R₂₉, R₃₀, R₃₁, R₃₄, and R₃₅ is a substituent. More preferably, at least one of R₂₂, R₂₃, R₂₇, R₃₀, and R₃₅ is a substituent. Still more preferably, at least one of R₂₂, R₂₇, R₃₀, R₃₁, and R₃₅ is a substituent. Further preferably, at least either R₂₇ or R₃₅ is a substituent. Particularly preferably, R₂₇ and R₃₅ are substituents. Preferred substituents include alkyl groups with 1 to 10 carbon atoms, aryl groups with 6 to 12 carbon atoms, and heteroaryl groups with 3 to 12 carbon atoms, more preferably alkyl groups with 1 to 4 carbon atoms and aryl groups with 6 to 12 carbon atoms, particularly tert-butyl, isopropyl, and phenyl groups.

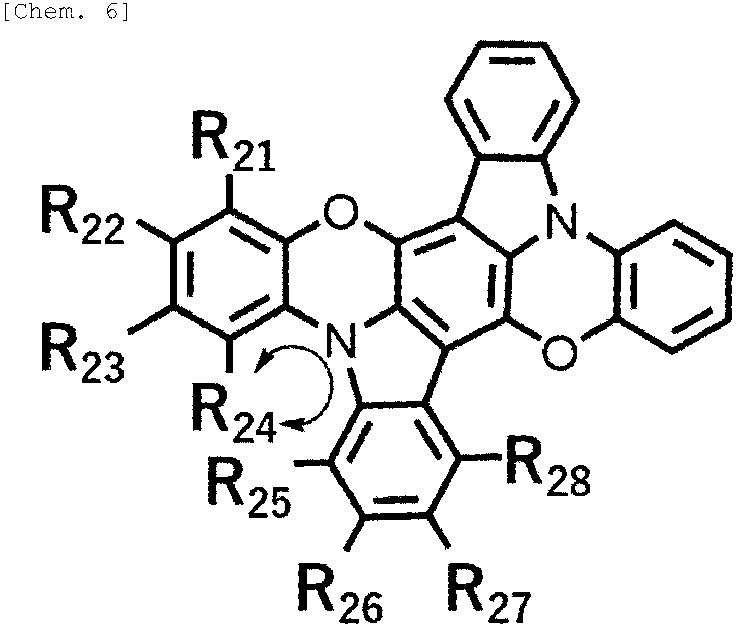

### [Table 3]

**Table 3**

| | R₂₁ | R₂₂ | R₂₃ | R₂₄ | R₂₅ | R₂₆ | R₂₇ | R₂₈ |
|---|---|---|---|---|---|---|---|---|
| Basic skeleton twist angle (calc.) | 12 | 7 | 6 | 26 | 27 | 9 | 7 | 16 |
| S1 (calc.) / eV | 2.84 | 2.84 | 2.82 | 2.88 | 2.86 | 2.74 | 2.83 | 2.74 |

For calculating the S1 energy and the basic skeleton twist angle, a molecular orbital calculation was used. In the molecular orbital calculation method, density functional theory (DFT), which is currently widely used, is used. The functional used was B3LYP, and the basis function was 6-31G*. The molecular orbital calculation method was performed using a currently widely used software program, Gaussian 09 (Gaussian 09, Revision C. 01, M. J. Frisch, G. W. Trucks, H. B. Schlegel, G. E. Scuseria, M. A. Robb, J. R. Cheeseman, G. Scalmani, V. Barone, B. Mennucci, G. A. Petersson, H. Nakatsuji, M. Caricato, X. Li, H. P. Hratchian, A. F. Izmaylov, J. Bloino, G. Zheng, J. L. Sonnenberg, M. Hada, M. Ehara, K. Toyota, R. Fukuda, J. Hasegawa, M. Ishida, T. Nakajima, Y. Honda, O. Kitao, H. Nakai, T. Vreven, J. A. Montgomery, Jr., J. E. Peralta, F. Ogliaro, M. Bearpark, J. J. Heyd, E. Brothers, K. N. Kudin, V. N. Staroverov, T. Keith, R. Kobayashi, J. Normand, K. Raghavachari, A. Rendell, J. C. Burant, S. S. Iyengar, J. Tomasi, M. Cossi, N. Rega, J. M. Millam, M. Klene, J. E. Knox, J. B. Cross, V. Bakken, C. Adamo, J. Jaramillo, R. Gomperts, R. E. Stratmann, O. Yazyev, A. J. Austin, R. Cammi, C. Pomelli, J. W. Ochterski, R. L. Martin, K. Morokuma, V. G. Zakrzewski, G. A. Voth, P. Salvador, J. J. Dannenberg, S. Dapprich, A. D. Daniels, O. Farkas, J. B. Foresman, J. V. Ortiz, J. Cioslowski, and D. J. Fox, Gaussian, Inc., Wallingford CT, 2010). The same method was used for molecular orbital calculations described hereinafter.

(1-4) At least either R₂₁ or R₂₉ of general formulas (2) and (3) is a substituent, thereby reducing concentration quenching. Since the basic skeleton is highly planar, when the compound is in a thin-film state, intermolecular stacking is likely to occur, thereby causing concentration quenching in the emission. In general formulas (2) and (3) in which at least either R₂₁ or R₂₉ is a substituent, the basic skeleton and the substituent are twisted in relative position by steric hindrance. This reduces the likelihood of intermolecular stacking in a thin-film state to reduce concentration quenching in emission. Accordingly, at least either R₂₁ or R₂₉ is preferably a substituent, and more preferably, R₂₁ and R₂₉ are substituents. Preferred substituents include alkyl groups with 1 to 10 carbon atoms, aryl groups with 6 to 12 carbon atoms, and a fluorine group, more preferably alkyl groups with 1 to 4 carbon atoms and aryl groups with 6 to 12 carbon atoms, particularly tert-butyl, isopropyl, and ortho-substituting phenyl groups.

The organic compounds represented by general formula (2) have the following features:
(1-5) The organic compounds represented by general formula (2), whose basic skeleton is constituted of six-membered rings, exhibit high T1 (lowest excited triplet energy).

Among the organic compounds according to the present invention, the organic compounds represented by general formula (2) are less likely to elongate in conjugation length than the organic compounds represented by the general formula (3) because X₃ and X₄ are not direct bonds. Therefore, the organic compounds represented by general formula (2) exhibit particularly higher T1 than the other organic compounds of the present invention. The organic compounds represented by general formula (2) are suitably used as a host material or an assist material in the organic light-emitting element because of such a feature. The organic compounds represented by general formula (2) are suitably used as a host material of the blue or green luminescent layer in the organic light-emitting element.

Specific examples of the organic compounds of the present invention are presented below. However, the present invention is not limited to the following compounds.

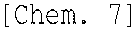

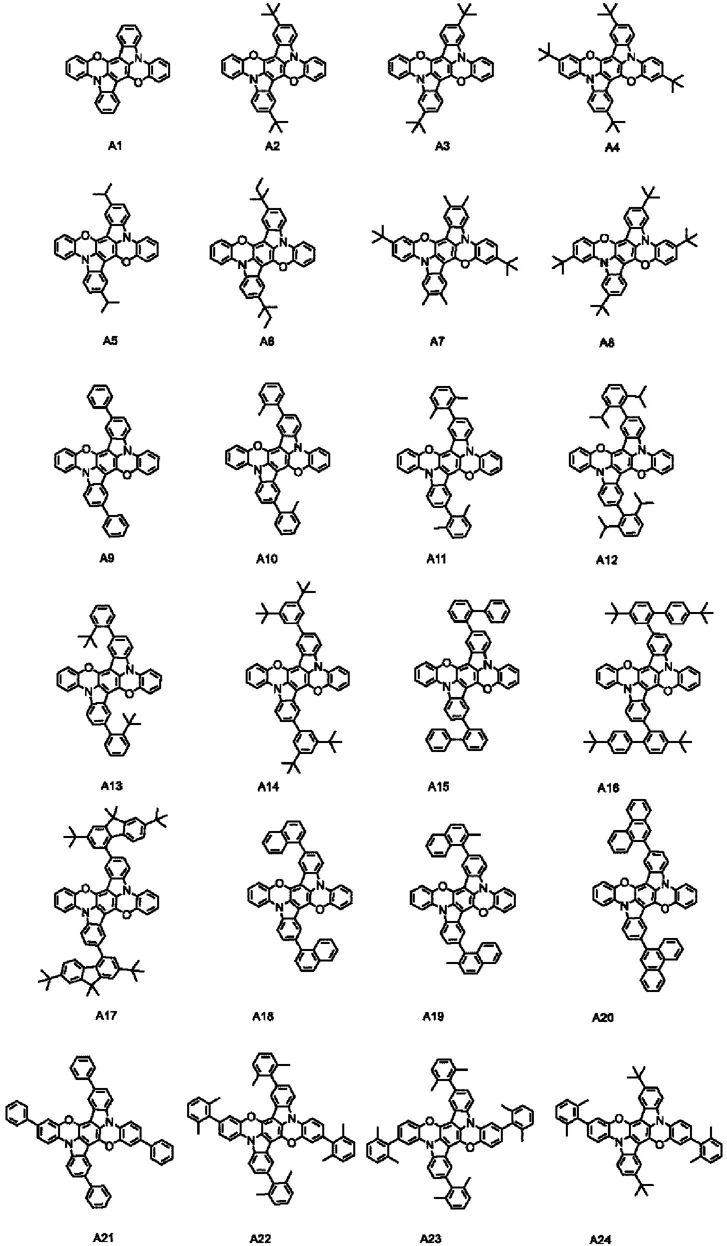

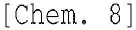

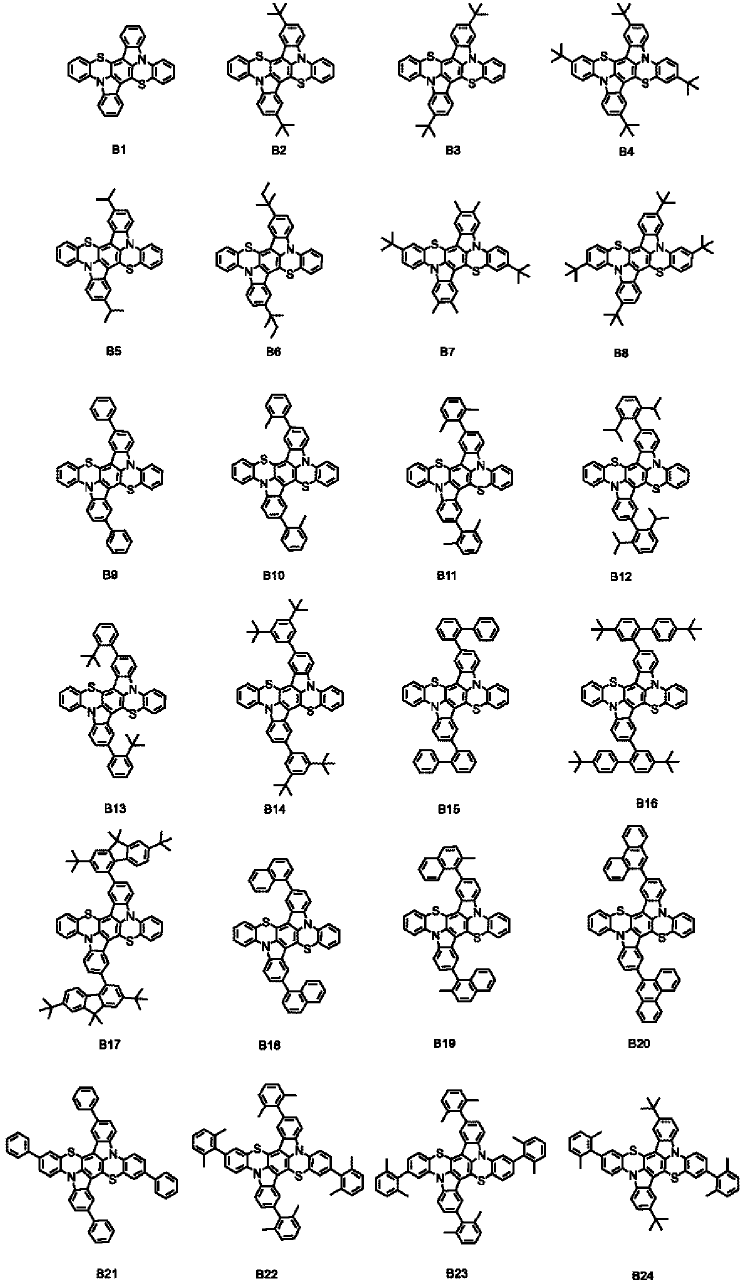

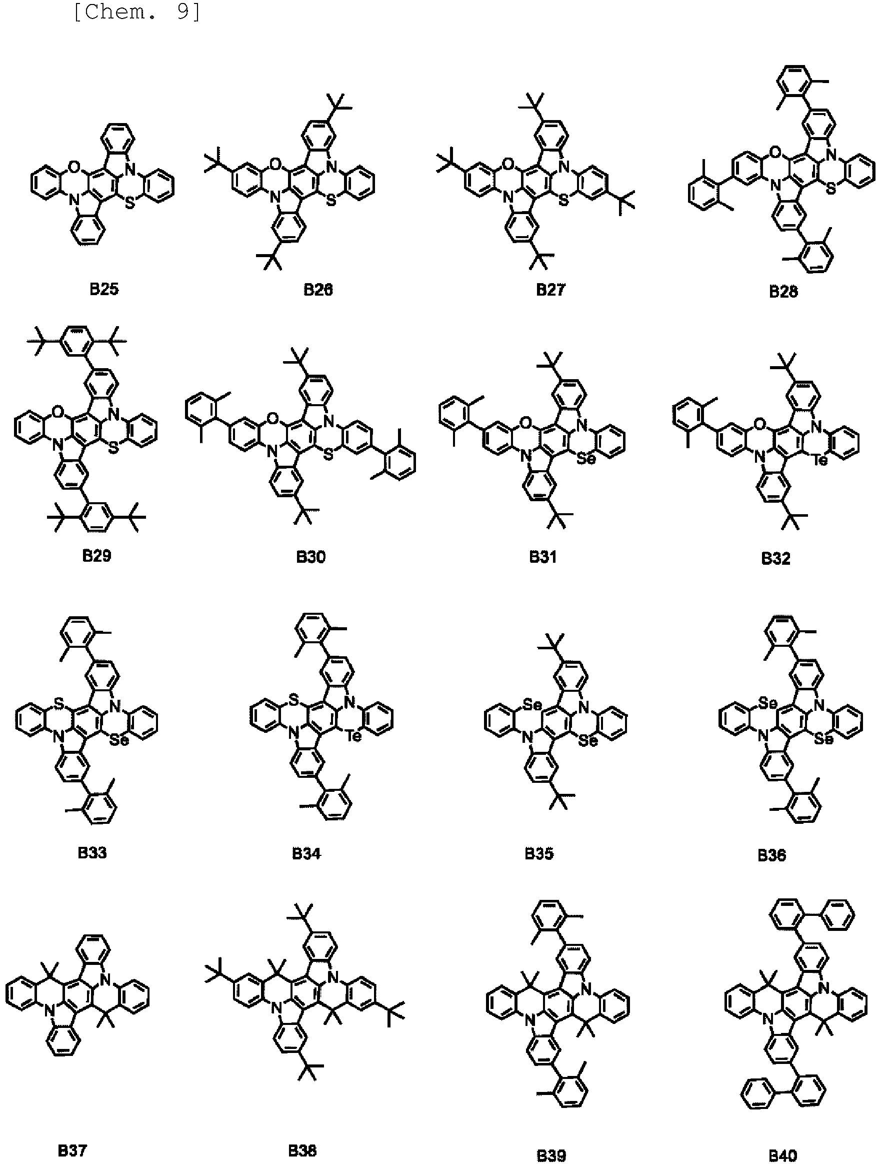

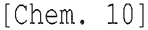

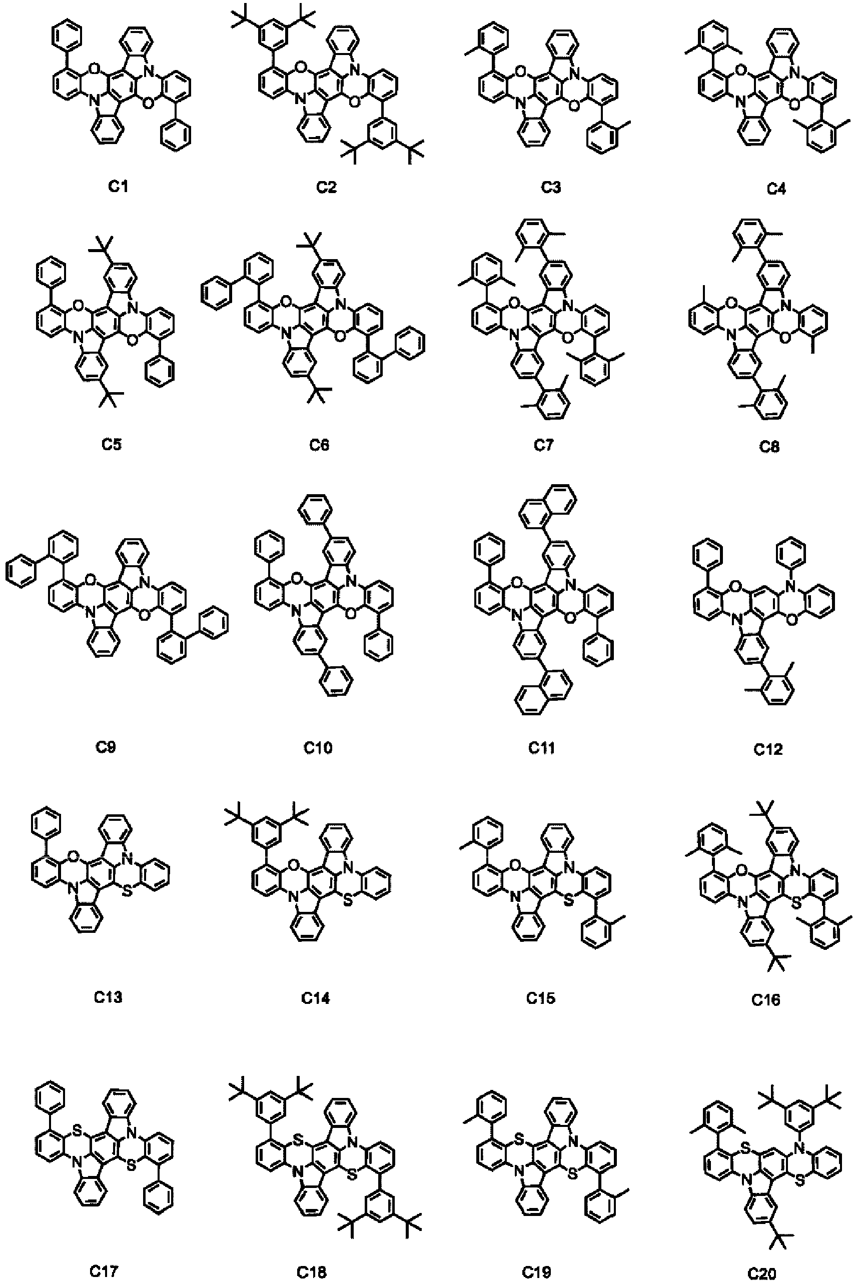

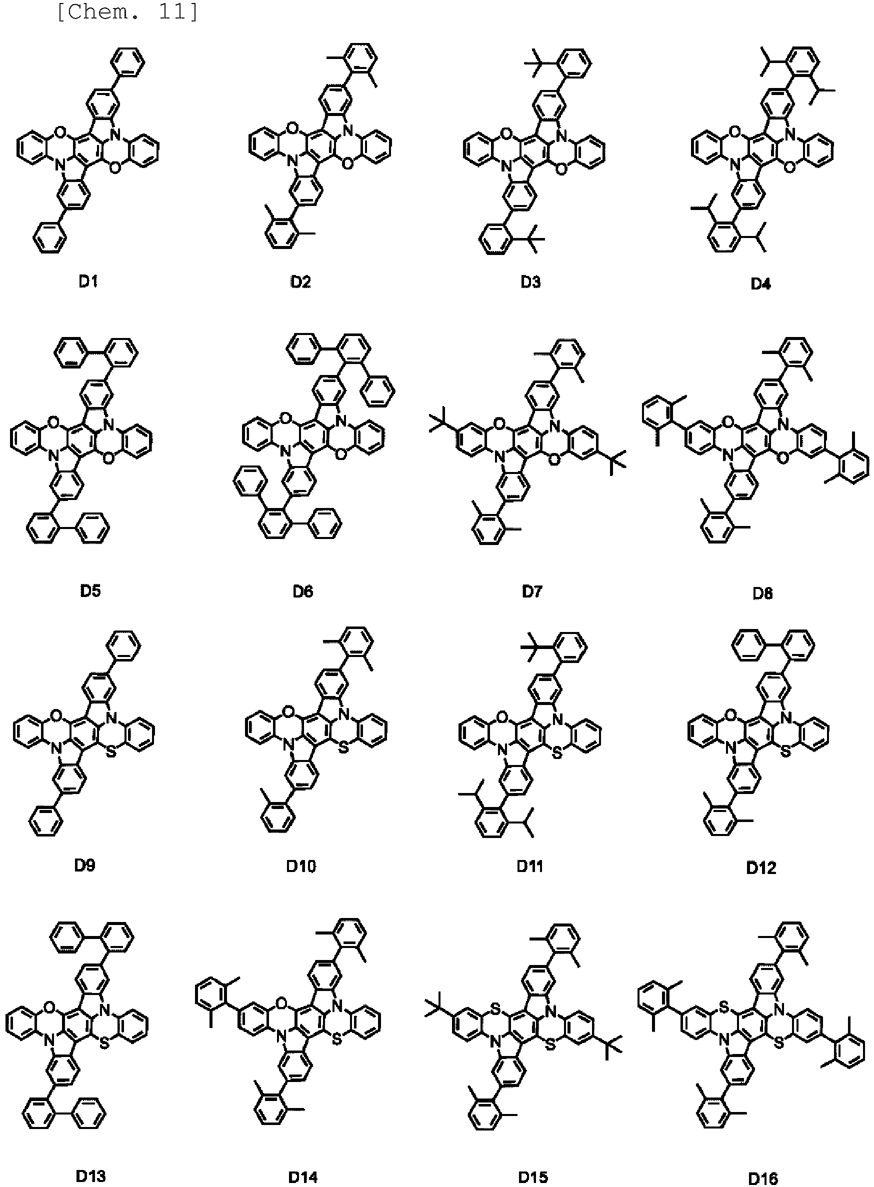

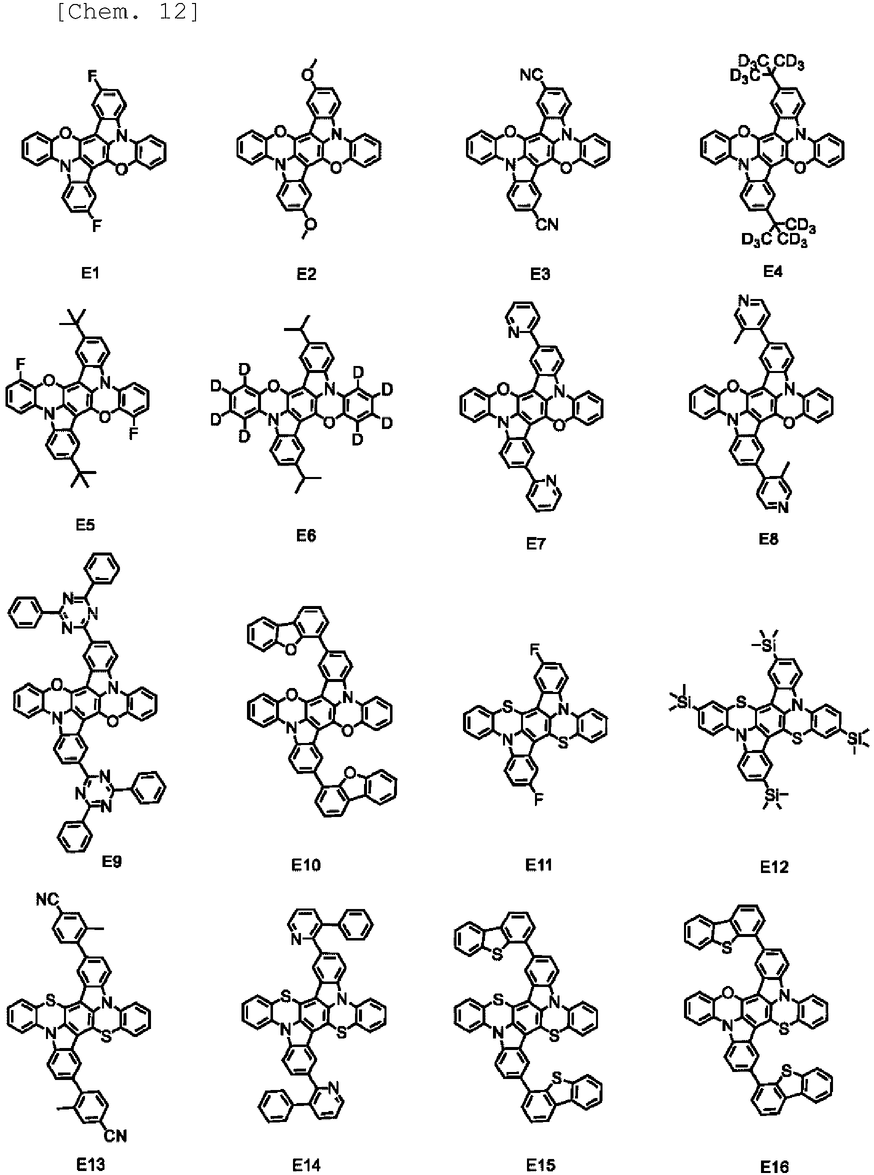

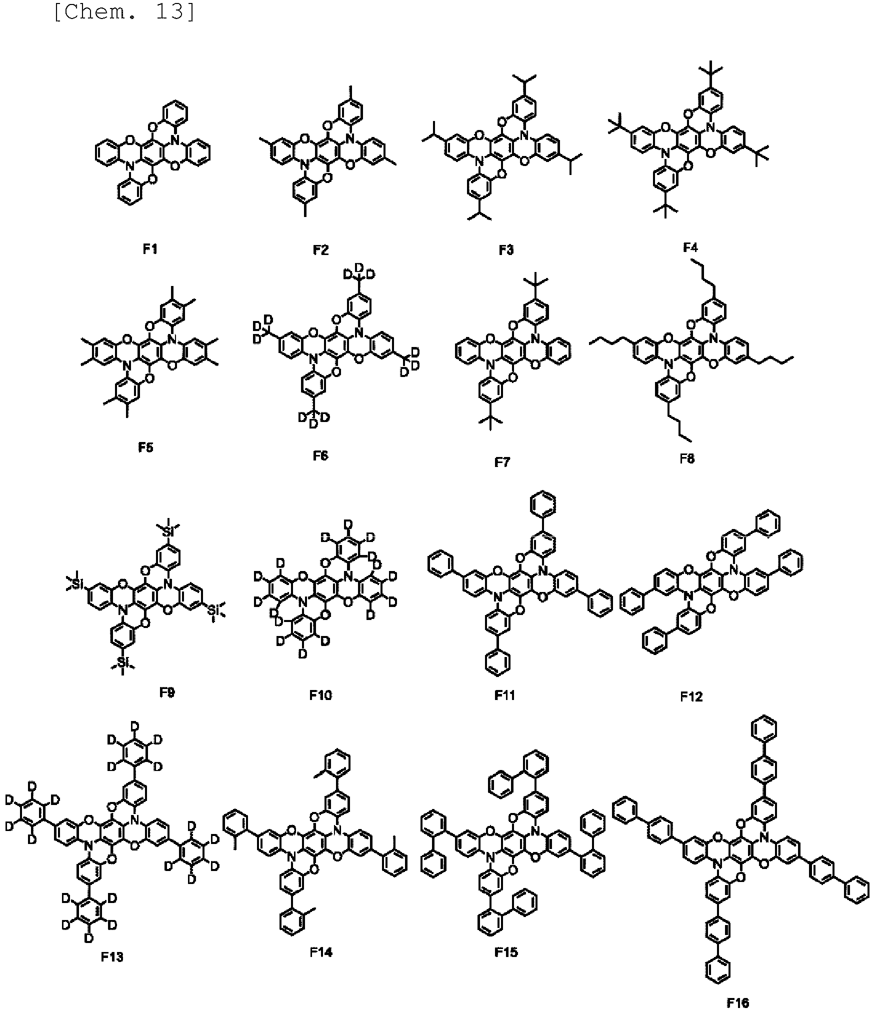

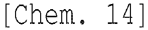

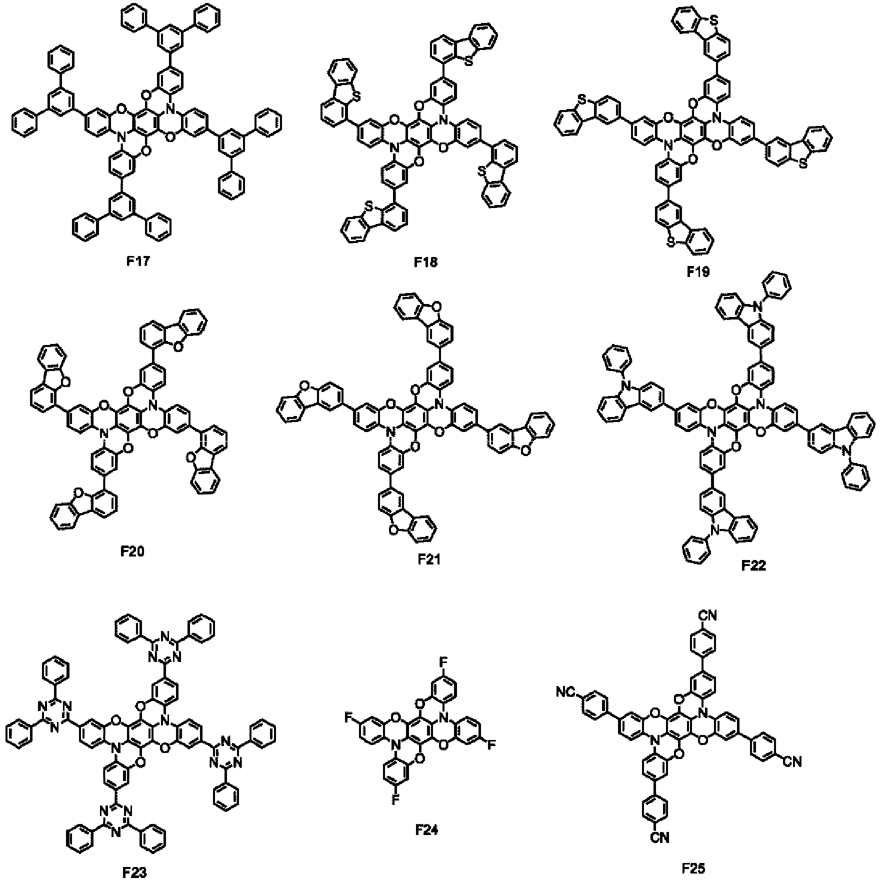

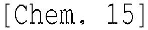

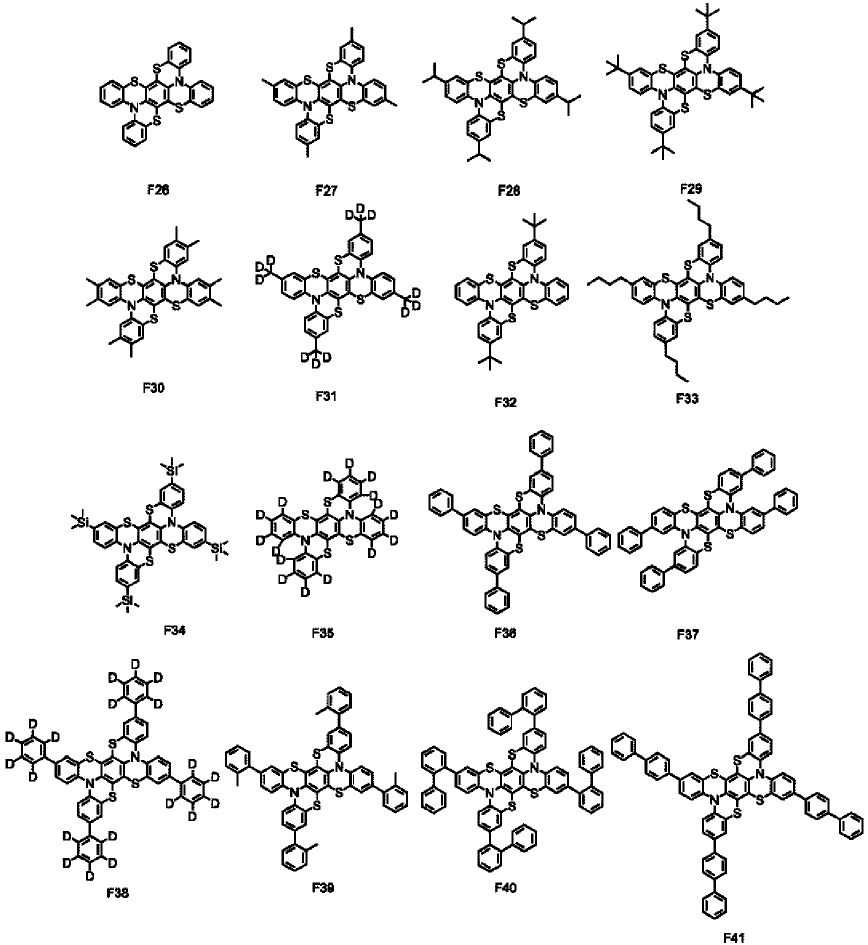

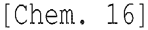

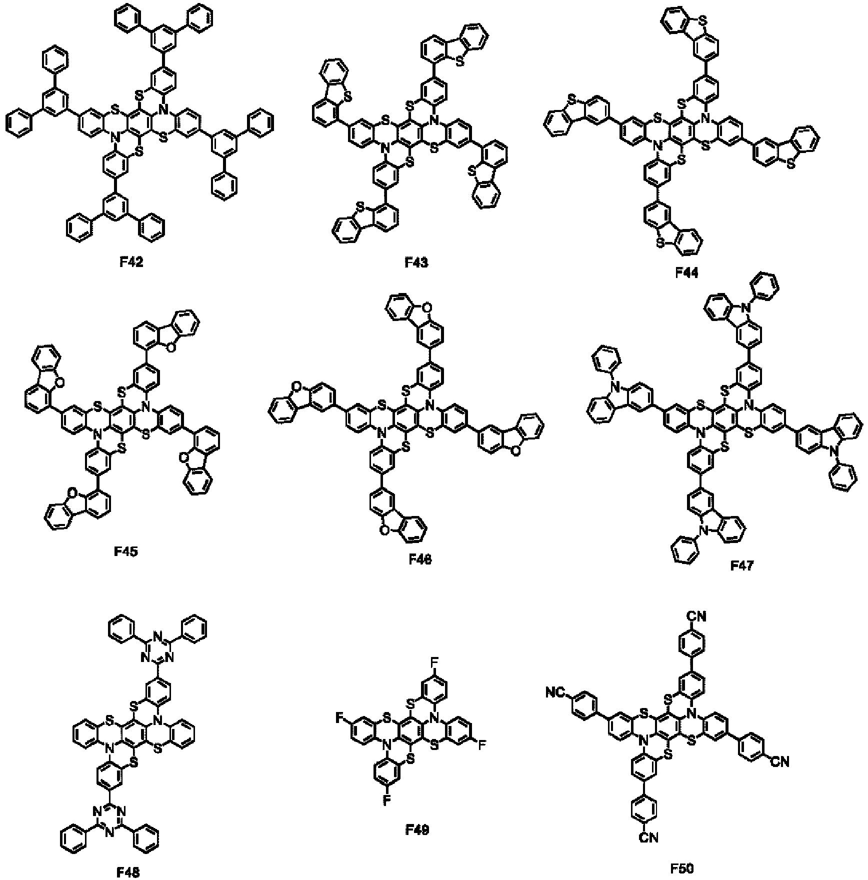

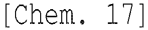

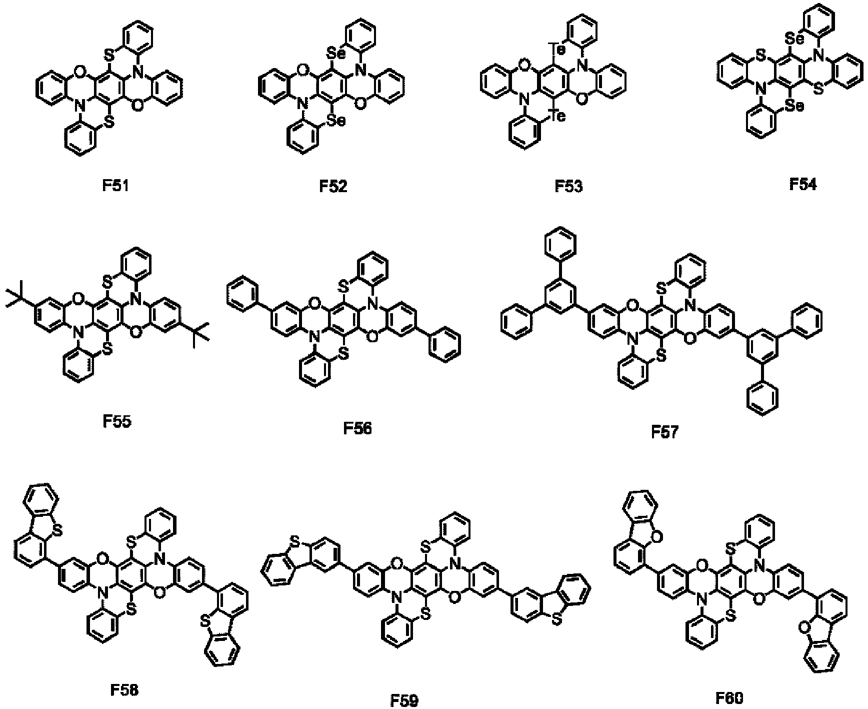

The exemplified compounds belonging to group A are compounds of general formula (3) in which at least one of R₂₂, R₂₃, R₂₇, R₃₀, R₃₁, and R₃₅ is a substituent and X₁ and X₂ are oxygen. Among the organic compounds according to the present invention, the group A compounds have basic skeletons with higher planarity than the compounds in the other groups, accordingly emitting light with the shortest wavelength and with high color purity.

The exemplified compounds belonging to group B, except for B37 to B40, are compounds of general formula (3) in which at least one of R₂₂, R₂₃, R₂₇, R₃₀, R₃₁, and R₃₅ is a substituent and X₁ and X₂ are any of oxygen, sulfur, selenium, and tellurium atoms. Among the organic compounds according to the present invention, the group B compounds have low HOMO and LUMO (far from the vacuum level) and produce the effect of being stable to oxidation.

Among the exemplified compounds belonging to group B, the organic compounds represented by B37 to B40 are compounds of general formula (1) in which X₁ or X₂ is a methylene group. When X₁ or X₂ is a methylene group, molecular association can be reduced. Accordingly, such compounds are less likely to cause concentration quenching.

The exemplified compounds belonging to group C, except for C12 and C20, are organic compounds of general formula (3) in which at least either R₂₁ or R₂₉ has a substituent. In the organic compounds belonging to group C among the organic compounds of the present invention, the basic skeleton and the substituents are twisted in relative position by steric hindrance, consequently reducing the likelihood of intermolecular stacking in a thin-film state to produce the effect of reducing concentration quenching.

C12 and C20 of the exemplified compounds belonging to group C are compounds of general formula (1) in which either pair of R₉ and R₁₀ or R₁₉ and R₂₀ are bound to form a fused ring. C12 and C20 produce the effect of emitting short wavelength light.

The exemplified compounds belonging to group D are compounds represented by general formula (3) in which R₂₆ and R₃₄ are substituents. Among the organic compounds according to the present invention, the compounds belonging to group D, in which R₂₆ and R₃₄ are substituents, are excellent in durability. Also, since the substituents are aryl groups, these compounds have high planarity and provide excellent color purity.

Exemplified compounds belonging to group E contain halogen atoms, deuterium atoms, silyl groups, alkoxy groups, cyano groups, or heteroaryl groups as substituents. Among the organic compounds according to the present invention, the group E compounds produce the effect of allowing the HOMO and LUMO to be adjusted.

The exemplified compounds belonging to group F are compounds represented by general formula (2). The exemplified compounds belonging to group F, whose basic skeleton is constituted of six-membered rings, exhibit high T1.

### (2) Details of Organic Light-Emitting Element

The organic light-emitting element disclosed herein will now be described. The organic light-emitting element disclosed herein at least includes a first electrode, a second electrode, and an organic compound layer between the electrodes. One of the first and second electrodes is an anode, and the other is a cathode. In the organic light-emitting element according to an embodiment, the organic compound layer may be composed of a single layer or have a multilayer structure including a plurality of layers, provided that the organic compound layer includes a luminescent layer. When the organic compound layer has a multilayer structure including a plurality of layers, the organic compound layer may include, a hole injection layer, a hole transport layer, an electron blocking layer, a hole/exciton blocking layer, an electron transport layer, and an electron injection layer, in addition to the luminescent layer. The luminescent layer may be composed of a single layer or have a multilayer structure including a plurality of layers.

In the organic light-emitting element disclosed herein, at least one layer of the above-mentioned organic compound layers contains an organic compound according to the present invention. More specifically, the organic compound of the present invention is contained in any of the above-mentioned luminescent layer, hole injection layer, hole transport layer, electron blocking layer, hole/exciton blocking layer, electron transport layer, and electron injection. Preferably, the organic compound of the present invention is contained in the hole transport layer, the electron blocking layer, the hole/exciton blocking layer, the electron transport layer, or the luminescent layer, particularly preferably in the luminescent layer.

In an embodiment of the organic light-emitting element in which the organic compound of the present invention is contained in the luminescent layer, the luminescent layer may further contain a first compound (hereinafter also referred to as a host material). The first compound has a higher S1 than the organic compound of the present invention. Also, the luminescent layer may further contain a second compound (hereinafter also referred to as an assist material). Preferably, the second compound has an S1 higher than the organic compound of the present invention and lower than the first compound.

In an embodiment of the organic light-emitting element in which the organic compound of the present invention is contained in the luminescent layer, the luminescent layer may be made of only the organic compound of the present invention or the organic compound of the present invention and other compounds. When the luminescent layer is made of the organic compound of the present invention and other compounds, the organic compound of the present invention may be used as the host material, the guest material, or a guest material in the luminescent layer. When the organic compound of the present invention is used as the host material, the luminescent layer may contain a third compound (hereinafter also referred to as the "guest material" or "dopant material"). Preferably, the third compound has a T1 lower than the S1 of the organic compound of the present invention and further preferably has an S1 lower than the S1 of the organic compound of the present invention. When the organic compound of the present invention is used as the assist material, the luminescent layer may contain a first compound and a third compound. The host used here refers to the compound accounting for the highest percentage of the total mass of the compounds in the luminescent layer. The assist material refers to a compound in a lower percentage by mass than the host in the luminescent and helps the guest emit light. The assist material is also called the second host. Also, the guest used here refers to a compound in a lower percentage by mass than the host in the luminescent layer and is responsible for the main luminescence.

When the organic compound of the present invention is used as the guest in the luminescent layer, the amount of the guest is preferably 0.01% to 30% by mass, more preferably 2% to 20% by mass, relative to the total mass of the luminescent layer.

The present inventors have found through various studies that using the organic compound of the present invention as the host or the guest, particularly as the guest, in the luminescent layer can provide an element that efficiently emits light with high luminance and has extremely high durability. The luminescent layer may be composed of a single layer or may have a multilayer structure. Also, the luminescent layer may contain another luminescent material exhibiting another emission color to mix red emission, which is the emission color of the embodiments of the invention. The multilayer structure in this case refers to a state where different luminescent layers are stacked one on top of another. In this instance, the emission color of the organic light-emitting element is not limited to red. For example, the emission color may be white or intermediate color. When the emission color is white, another luminescent layer emits a color light other than red light, such as blue or green. The luminescent layer is formed by vapor deposition or coating. This process will be detailed later in the Examples described later.

The organic compound of the present invention may be used as a constituent of other organic compound layers of the organic light-emitting element disclosed herein, apart from the luminescent layer. For example, the organic compound may be used as a constituent of the electron transport layer, the electron injection layer, the hole transport layer, the hole injection layer, the hole blocking layer, or the like. In this instance, the emission color of the organic light-emitting element is not limited to red. For example, the emission color may be white or intermediate color.

The organic compound of the present invention may be used in combination with one or more known low-molecular-weight or polymeric compounds used as a hole injecting or a hole transporting material, a compound acting as a host, a luminescent material, an electron injecting or an electron transporting material, and the like, if necessary. Examples of these compounds are presented below.

As the hole injecting or transporting material, materials with high hole mobility are preferred to facilitate the injection of holes from the anode and to transport the injected holes to the luminescent layer. Also, materials with a high glass transition temperature are preferred to reduce the crystallization or any other deterioration of the layer in the organic light-emitting element. Low-molecular-weight or polymeric hole injecting or transporting materials include triarylamine derivatives, aryl carbazole derivatives, phenylenediamine derivatives, stilbene derivatives, phthalocyanine derivatives, porphyrin derivatives, poly(vinyl carbazole), poly(thiophene), and other electrically conductive polymers. Such a hole injecting or transporting material is also suitably used in the electron blocking layer. Examples of the compounds that can be used as the hole injecting or transporting material include, but are not limited to, the following.

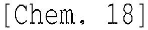

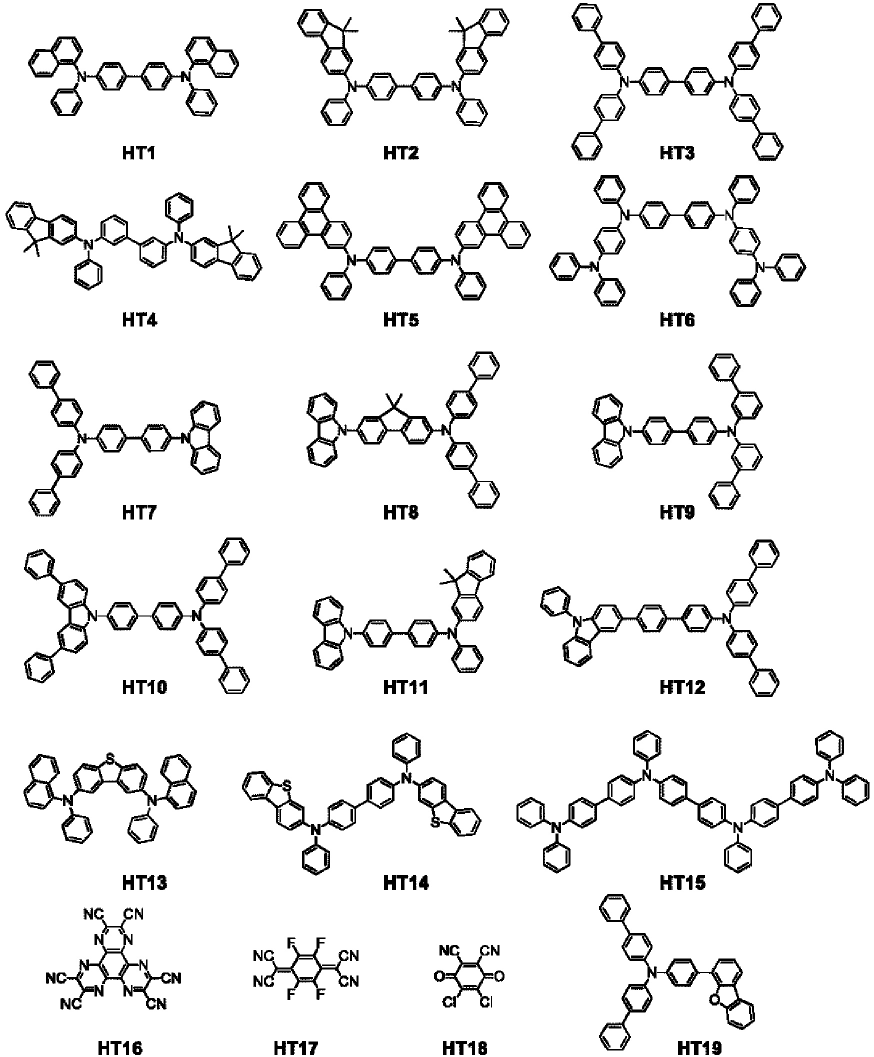

Among the above-presented hole transporting materials, HT16 to HT18 can reduce the driving voltage when used in the layer in contact with the anode. HT16 is widely used in organic light-emitting elements. HT2, HT3, HT4, HT5, HT6, HT10, or HT12 may be used in the organic compound layer in contact with HT16. A plurality of materials may be used in one organic compound layer.

Luminescent materials mainly involved in light emission include fused ring compounds (such as fluorene derivatives, naphthalene derivatives, pyrene derivatives, perylene derivatives, tetracene derivatives, anthracene derivatives, and rubrene), quinacridone derivatives, coumarin derivatives, stilbene derivatives, organoaluminum complexes such as tris(8-quinolinolato) aluminum, iridium complexes, platinum complexes, rhenium complexes, copper complexes, europium complexes, ruthenium complexes, and polymer derivatives such as poly(phenylene vinylene) derivatives, polyfluorene derivatives, and polyphenylene derivatives.

Examples of compounds that can be used as the luminescent material include, but are not limited to, the following.

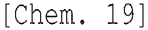

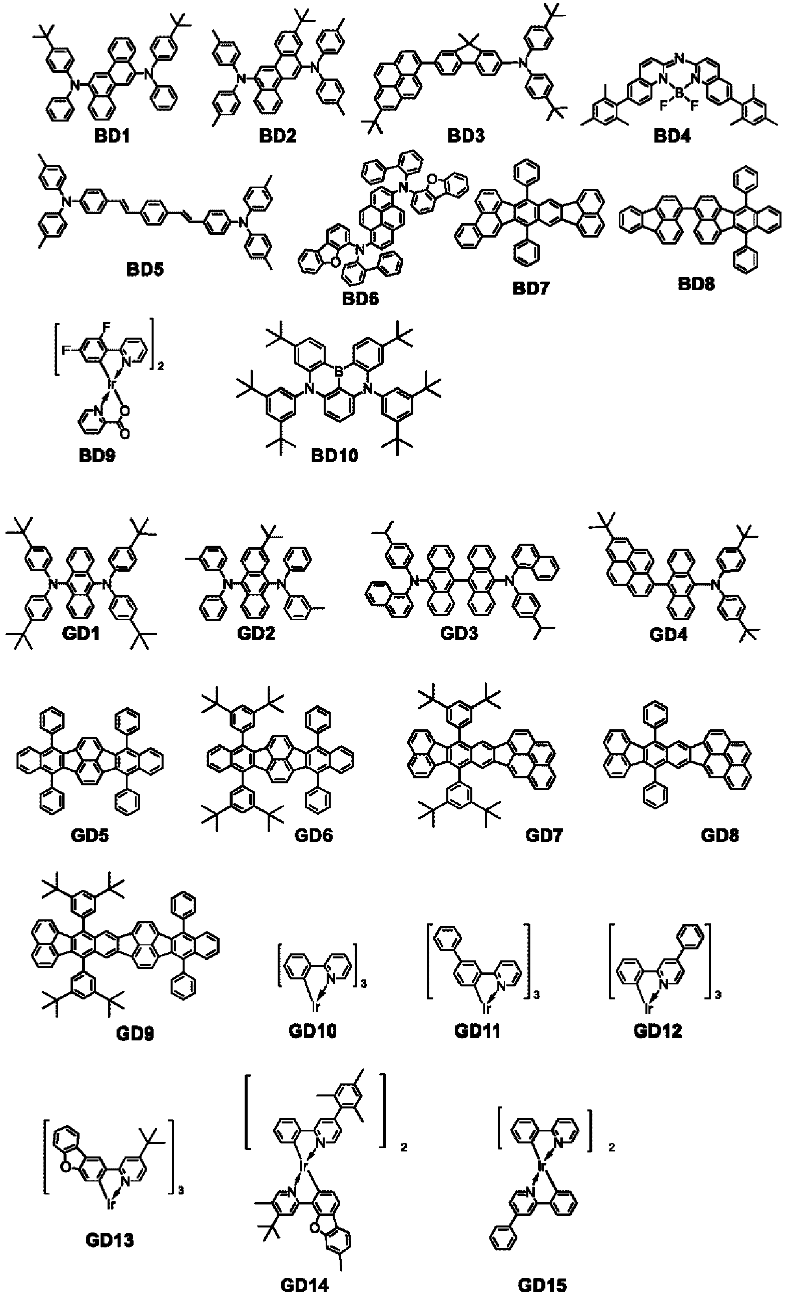

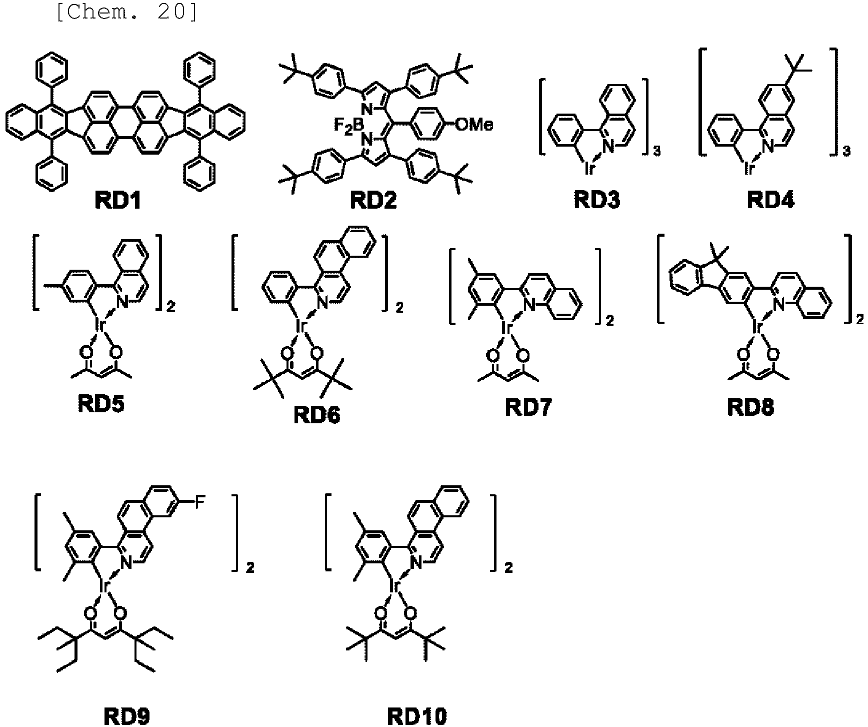

Luminescent materials that are hydrocarbon compounds are preferred because such compounds can reduce the decrease of luminous efficiency caused by exciplex formation and the degradation of color purity caused by changes of the emission spectrum of the luminescent material resulting from exciplex formation.

Hydrocarbon compounds are made up only of carbon and hydrogen. Among the above exemplified compounds, BD7, BD8, GD5 to GD9, and RD1 correspond to hydrocarbon compounds.

Luminescent materials defined by a fused polycyclic structure containing a 5-membered ring are preferred because such compounds have high ionization potential and are less likely to oxidize, accordingly leading to long-life elements with high durability. Among the above-exemplified compounds, BD7, BD8, GD5 to GD9, and RD1 correspond to such compounds.

Examples of the luminescent layer host or assist material contained in the luminescent layer include aromatic hydrocarbon compounds and their derivatives, carbazole derivatives, dibenzofuran derivatives, dibenzothiophene derivatives, organoaluminum complexes such as tris(8-quinolinolato) aluminum, and organoberyllium complexes.

Exemplary compounds that can be used as the luminescent layer host or assist material include, but are not limited to, the following.

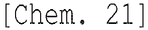

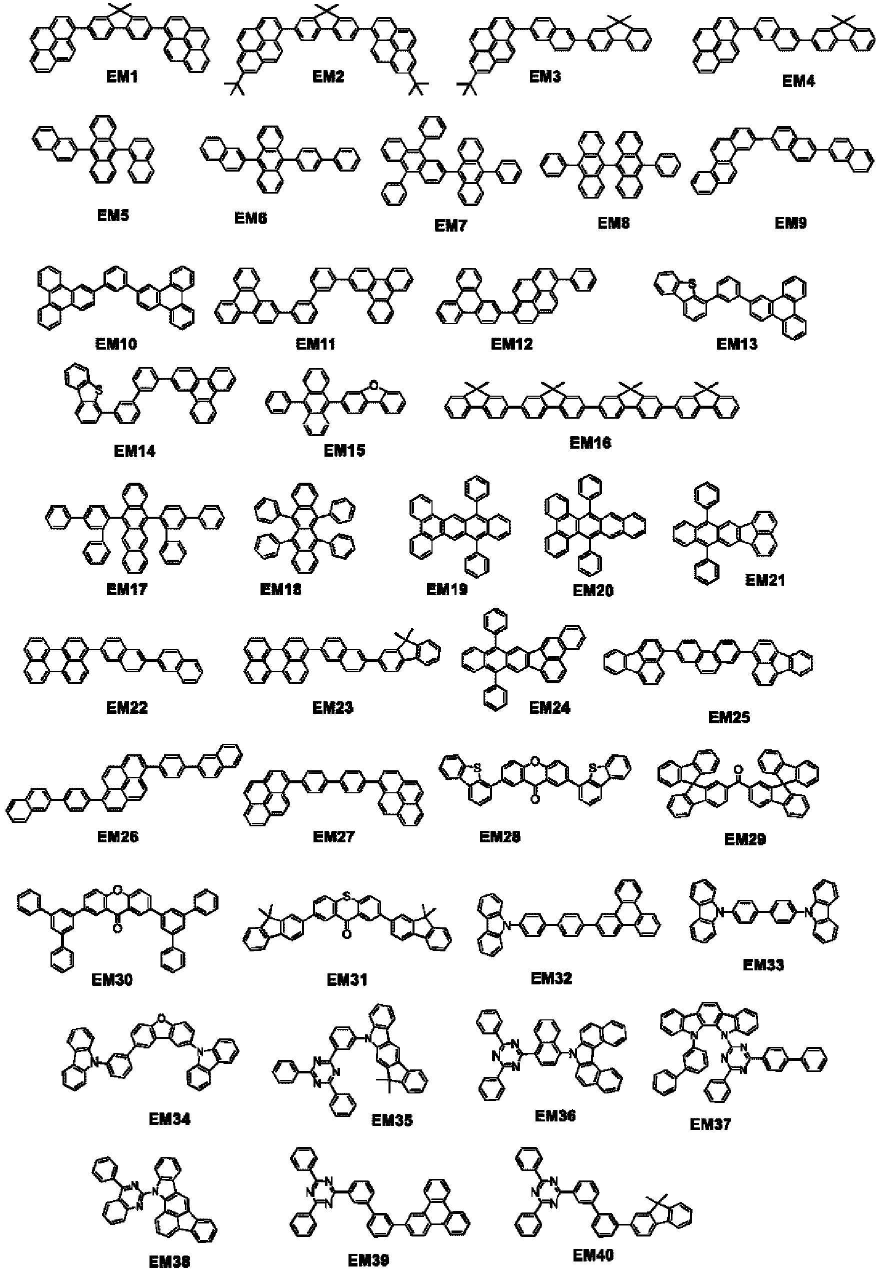

Host materials that are hydrocarbon compounds allow the compound of the present invention to easily trap electrons and holes to enhance the effect of increasing efficiency. Hydrocarbon compounds are made up only of carbon and hydrogen. Among the above exemplified compounds, EM1 to EM12 and EM16 to 27 correspond to hydrocarbon compounds. The host material preferably contains an aryl group with 6 to 18 carbon atoms and is further preferably a hydrocarbon compound. When the organic compound of the present invention is used as an assist material, the host material preferably has at least one of a pyrene skeleton, a triphenylene skeleton, and a chrysene skeleton. Among the above exemplified compounds, EM1 to 4, EM9 to 12, EM26, and EM27 are such compounds.

The electron transporting material can be selected arbitrarily from the compounds capable of transporting electrons injected from the cathode to the luminescent layer and is selected in view of, for example, the balance with the hole mobility of the hole transporting material. Materials capable of transporting electron include oxadiazole derivatives, oxazole derivatives, pyrazine derivatives, triazole derivatives, triazine derivatives, quinoline derivatives, quinoxaline derivatives, phenanthroline derivatives, organoaluminum complexes, and fused ring compounds (e.g., fluorene derivatives, naphthalene derivatives, chrysene derivatives, anthracene derivatives, etc.) The above electron transporting materials are also suitably used in the hole blocking layer.

Examples of the compounds that can be used as the electron transporting material include, but are not limited to, the following:

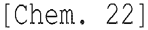

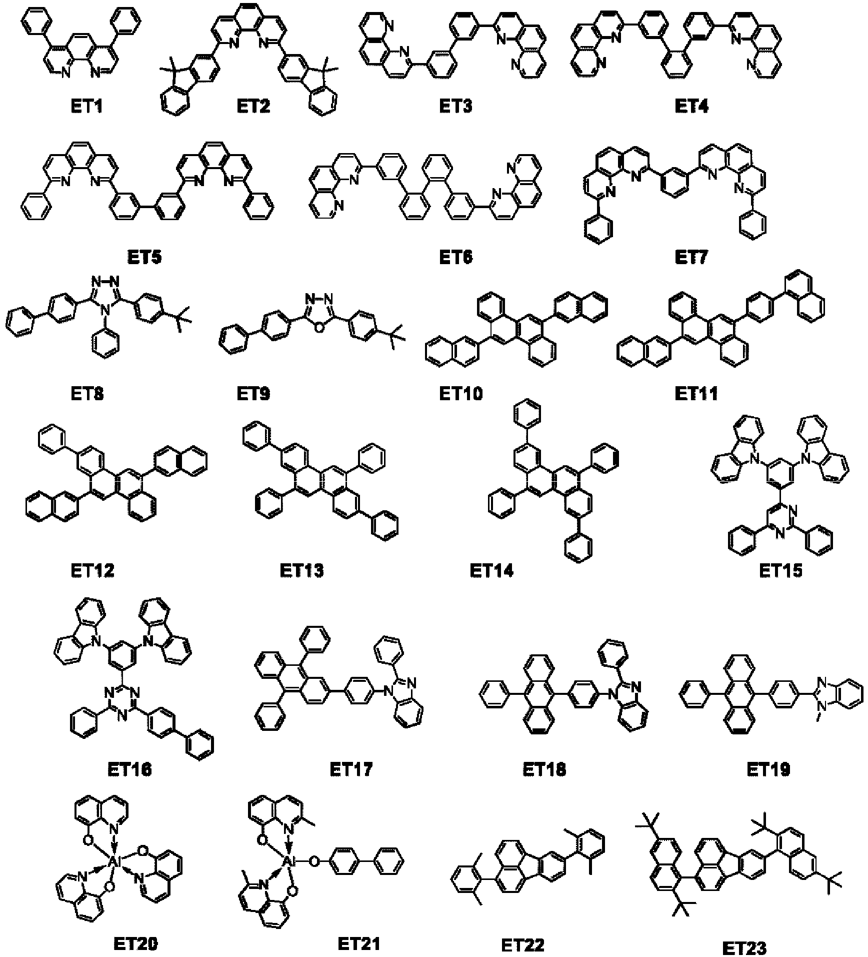

The electron injecting material can be selected arbitrarily from the compounds that can facilitate the injection of electrons from the cathode and is selected in view of, for example, the balance with hole injection. Organic n-type or reducing dopants can also be use. Examples include alkali metal-containing compounds such as lithium fluoride, lithium complexes such as lithium quinolinol, benzoimidazolidene derivatives, imidazolidene derivatives, fulvalene derivatives, and acridine derivatives.

Also, the electron transporting material may be combined.

### [Structure of Organic Light-Emitting Element]

The organic light-emitting element is provided by forming an insulating layer, a first electrode, one or more organic compound layers, and a second electrode on a substrate. A protective layer, a color filter, a microlens, or the like may be provided over the cathode. For the color filter, if provided, a planarizing layer may be disposed between the protective layer and the color filter. The planarizing layer may be made of, for example, acrylic resin. The same applies when a planarizing layer is disposed between the color filter and the microlens.

### [Substrate]

The substrate may be made of quartz, glass, silicon wafer, resin, metal, or the like. A switching element, such as a transistor, and wires may be arranged on the substrate, and over which an insulating layer is formed. The insulating layer may be made of any material, provided that contact holes can be formed in the insulating layer for wiring to the first electrode, and that insulation can be ensured from wiring lines not being connected. For example, polyimide or similar resin, silicon oxide, silicon nitride, or the like may be used.

### [Electrodes]

A pair of electrodes may be used as electrodes. The pair of electrodes may be an anode and a cathode. In the organic light-emitting element to which an electric field is applied in the light-emitting direction, the electrode with a higher potential is the anode, and the other is the cathode. Also, the electrode that supplies holes to the luminescent layer is the anode, and the electrode that supplies electrons is the cathode.

The anode is desirably made of a material having as high a work function as possible. Examples of such a material include simple metals, such as gold, platinum, silver, copper, nickel, palladium, cobalt, selenium, vanadium, and tungsten, mixtures containing simple metals or alloys formed by combining those metals, and metal oxides, such as tin oxide, zinc oxide, indium oxide, indium tin oxide (ITO), and zinc indium oxide. Electrically conductive polymers, such as polyaniline, polypyrrole, and polythiophene, may also be used.

These electrode materials may be used individually or in combination. The anode may be composed of a single layer or a plurality of layers.

For a reflection electrode, for example, chromium, aluminum, silver, titanium, tungsten, molybdenum, or an alloy or multilayer composite of these metals may be used. These materials can also function as a reflection film that does not serve as an electrode. For a transparent electrode, an electrically conductive transparent metal oxide layer of, for example, indium tin oxide (ITO) or indium zinc oxide, but not limited to, may be used. Photolithography technology can be used to from the electrodes.

The cathode is desirably made of a material having a low work function. Examples of the cathode material include alkali metals, such as lithium; alkaline-earth metals, such as calcium; other simple metals, such as aluminum, titanium, manganese, silver, lead, and chromium; and mixtures containing these simple metals. Alloys formed by combining these simple metals may be used. Examples of such an alloy include magnesium-silver, aluminum-lithium, aluminum-magnesium, silver-copper, and zinc-silver. A metal oxide, such as indium tin oxide (ITO), may be used. These electrode materials may be used individually or in combination. The cathode may be composed of a single layer or a plurality of layers. Preferably, silver is used, and silver alloy is further preferred because silver alloy reduces the aggregation of silver. Any proportions in the alloy are acceptable as long as the aggregation of silver can be reduced. For example, the ratio of silver to other metals may be 1:1 or 3:1.

The cathode may be defined by, but not limited to, an electrically conductive oxide layer, such as ITO, to provide a top emission element or a reflection electrode, such as aluminum (Al), to provide a bottom emission element. The cathode may be formed by any method without limitation, but direct current or alternating current sputtering is more suitable for film coverage and reducing resistance.

### [Organic Compound Layer]

The organic compound layer may be composed of a single layer or a plurality of layers. The plurality of layers may be called a hole injection layer, a hole transport layer, an electron blocking layer, a luminescent layer, a hole blocking layer, an electron transport layer, and an electron injection layer, depending on the function. The organic compound layer is mainly made of organic compounds but may contain inorganic atoms or inorganic compounds. For example, the organic compound layer may contain copper, lithium, magnesium, aluminum, iridium, platinum, molybdenum, zinc, or the like. The organic compound layer may be disposed between the first electrode and the second electrode and may be disposed in contact with the first and the second electrodes.

### [Protective Layer]

A protective layer may be provided over the cathode. For example, a glass sheet with a moisture absorbent may be bonded onto the cathode to reduce the penetration of water or the like into the organic compound layer, thus reducing the occurrence of display failure. In another embodiment, a passivation film of silicon nitride or the like may be provided over the cathode to reduce the penetration of water or the like into the organic compound layer. For example, after being formed, the cathode is transported to another chamber with a vacuum maintained, and a 2 um-thick silicon nitride film may be deposited by CVD, thus obtaining a protective layer. After deposition by CVD, a protective layer may be formed by atomic layer deposition (ALD). The material of the film formed by ALD may be, but is not limited to, silicon nitride, silicon oxide, aluminum oxide, or the like. A silicon nitride film may be further formed by CVD on the film formed by ALD. The thickness of the film formed by ALD may be smaller than the film formed by CVD. Specifically, it may be 500 or less, or even 100 or less.

### [Color Filter]

A color filter may be provided on the protective layer. For example, a color filter that allows for the size of the organic light-emitting element may be provided on another substrate, and the color filter and the substrate provided with the organic light-emitting element are bonded together. Alternatively, the color filter may be patterned on the above-described protective layer by photolithography technology. The color filter may be made of a polymer.

### [Planarizing Layer]

A planarizing layer may be disposed between the color filter and the protective layer. The planarizing layer is intended to reduce the unevenness of the underlying layer. Such a layer may be called a material resin layer so that the name does not limit the purpose. The planarizing layer may be made of an organic compound, which may be low molecular weight or polymeric but is preferably polymeric.

Planarizing layers may be provided on each of the upper and lower side of the color filter, and their material may be the same or different. Examples include polyvinylcarbazole resin, polycarbonate resin, polyester resin, ABS resin, acrylic resin, polyimide resin, phenol resin, epoxy resin, silicone resin, and urea resin.

### [Microlens]

The organic light-emitting device may include an optical member, such as a microlens, on the light emitting side. The microlens may be made of acrylic resin, epoxy resin, or the like. The microlens may be used to increase the amount of light extracted from the organic light-emitting device and to control the direction of the extracted light. The microlens may have a hemispherical shape. For a hemispherical microlens, the apex of the microlens is the contact point of the tangent line parallel to the insulating layer, among the tangent lines of the hemisphere, with the hemisphere. The apex of the microlens can be determined in the same manner in any sectional view. In other words, in a sectional view of a microlens, the contact point of the tangent line of the hemisphere parallel to the insulating layer, among the tangent lines of the microlens, with the hemisphere is the apex of the microlens.

Also, the midpoint of the microlens can be defined. In a cross-section of microlenses, the midpoint of an imaginary line segment from the point where an arc shape ends to the point where another arc shape ends can be called the midpoint of a microlens. The cross-section to determine the apex and the midpoint may be a cross-section perpendicular to the insulating layer.

### [Opposing Substrate]

An opposing substrate may be disposed on the planarizing layer. The opposing substrate is located opposing the foregoing substrate and is, therefore, called the opposing substrate. The opposing substrate may be made of the same material as the foregoing substrate. When the foregoing substrate is a first substrate, the opposing substrate may be a second substrate.

### [Organic Layer(s)]

The organic compound layers (hole injection layer, hole transport layer, electron blocking layer, luminescent layer, hole blocking layer, electron transport layer, electron injection layer, etc.) of the organic light-emitting element according to an embodiment may be formed by the following process.

The organic compound layers of the organic light-emitting element according to an embodiment may be formed in a dry process performed by, for example, vacuum deposition, ionized deposition, sputtering, or using plasma. As an alternative to the dry process, a wet process may be applied in which layers are formed by a known coating of a material dissolved in an appropriate solvent (e.g., spin coating, dipping, casting method, LB method, or ink jet method).

Layers formed by vacuum deposition, solution coating, or the like are less likely to crystallize and are thus stable over time. When a layer is formed by coating, an appropriate binder resin may be combined.

Examples of the binder resin include, but are not limited to, polyvinylcarbazole resin, polycarbonate resin, polyester resin, ABS resin, acrylic resin, polyimide resin, phenol resin, epoxy resin, silicone resin, and urea resin.

Such a binder resin may be used individually as a homopolymer or a copolymer, or two or more resins may be mixed. Other known additives, such as a plasticizer, an antioxidant, and an ultraviolet light adsorbent, may further be used if necessary.

### [Pixel Circuit]

A light-emitting device may include a pixel circuit connected to the light-emitting element. The pixel circuit may be a type of active matrix that independently controls a first light-emitting element and a second light-emitting element for emission. The active matrix circuit may be controlled by either voltage programming or current programming. The driving circuit includes a pixel circuit for each pixel. The pixel circuit may include a transistor to control the light-emitting element and the luminance of emission from the light-emitting element, a transistor to control emission timing, a capacitor to hold the gate voltage of the transistor to control the luminance of emission, and a transistor for connection to the GND but not via the light-emitting element.

The light-emitting device includes a display region and a peripheral region around the display region. The display region has pixel circuits, and the peripheral region has a display control circuit. In the transistor of the pixel circuit, the mobility may be lower than that in the transistor of the display control circuit.

The transistor of the pixel circuit may have current-voltage characteristics with a smaller slope than the transistor of the display control circuit. The slope of current-voltage characteristics can be measured using what is called Vg-Ig characteristics.

The transistor of the pixel circuit is the transistor connected to a light-emitting element, such as a first light-emitting element.

### [Pixel]

The organic light-emitting device has a plurality of pixels. Each pixel includes sub-pixels that emit different colors from each other. Sub-pixels may have their respective RGB emission colors.

The pixels emit light from a region called a pixel aperture. This region is the same as a first region. The pixel aperture may be 15 um or less and may be 5 um or more. More specifically, it may be 11 um, 9.5 um, 7.4 um, 6.4 um, and so forth.

The distance between adjacent sub-pixels may be 10 um or less, for example, 8 um, 7.4 um, or 6.4 um.

The pixels may be arranged in a known array in plan view. For example, the pixels may be arranged in striped array, a delta array, pentile array, or a Bayer array. The sub-pixels may have any known shape in plan view. For example, the shape may be quadrilateral, such as rectangular or rhombic, hexagonal, and so forth. Of course, the shape may not be an exact figure, and, for example, a shape close to a rectangle is considered rectangular. The shape of sub-pixels and the pixel arrangement may be combined.

### [Applications of Organic Light-Emitting Element]

The organic light-emitting element according to an embodiment of the present invention can be used as a component of a display device or a lighting device. In addition, the organic light-emitting element may be used as an exposure light source of an electrophotographic image forming apparatus, a backlight of a liquid crystal display device, or a light-emitting device including a white light source provided with a color filter.

The display device may be used in an image information processing apparatus that includes an image input section to which image information is input from an area CCD, a linear CCD, a memory card, or the like and an information processing section in which the inputted information is processed, and a display section on which the inputted information is displayed.

The display section of an imaging device or an ink jet printer may have a function as a touch panel. The touch panel function may be operated by, but not limited to, using infrared, a capacitive scheme, a resistive film, or electromagnetic induction. Also, the display device may be used as a display section of a multifunctional printer.

The display device according to an embodiment of the present invention will now be described with reference to drawings.

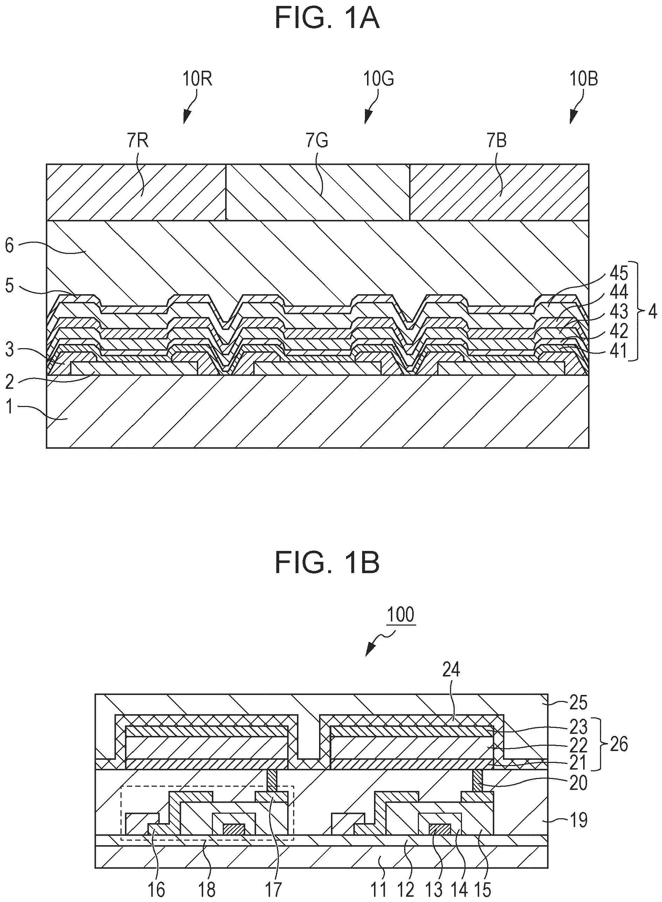

Figs. 1A and 1B are each a schematic sectional view of a display device including organic light-emitting elements and transistors connected to the respective organic light-emitting elements. The transistors are an example of active elements. The transistors may be thin-film transistors (TFTs) .

Fig. 1A depicts an example of a pixel that is a component of the display according to an embodiment of the present invention. The pixel includes sub-pixels 10. The sub-pixels are categorized into 10R, 10G, and 10B depending on the emission color. Emission colors may be identified by the wavelength of the light emitted from the luminescent layer, or color filters may selectively transmit the light emitted from the sub-pixels or convert the color of the light. Each sub-pixel includes, on an interlayer insulating layer 1, a reflection electrode 2, which is a first electrode, an insulating layer 3 covering the edge of the reflection electrode 2, an organic compound layer 4 covering the first electrode and the insulating layer, a transparent electrode 5, a protective layer 6, and a color filter 7.

Transistors and capacitor elements may be disposed under or within the interlayer insulating layer 1. The transistors and the respective first electrodes may be electrically coupled through a contact hole or the like not shown in the figure.

The insulating layer 3 is also called a bank or pixel isolation film. The insulating layer covers the edges of the first electrodes and surrounds the first electrodes. The portion of the first electrode not covered with the insulating layer is in contact with the organic compound layer 4 to act as a light-emitting region.

The organic compound layer 4 includes a hole injection layer 41, a hole transport layer 42, a first luminescent layer 43, a second luminescent layer 44, and an electron transport layer 45.

The second electrode 5 may be transparent, reflective, or semitransparent.

The protective layer 6 reduces water permeation into the organic compound layer. The protective layer, which is illustrated as a single layer, may include a plurality of layers. Each layer may include an inorganic compound layer or an organic compound layer.

Color filters 7 are categorized into 7R, 7G, and 7 B depending on the color. The color filters may be formed on a planarizing layer not shown in the figure. Also, the color filters may be provided with a resin protective layer, not shown in the figure, over the color filters. The color filters may be formed on the protective layer 6. Alternatively, the color filters may be formed on an opposing substrate such as a glass substrate and then bonded together.

In the display device 100 depicted in Fig. 1B, organic light-emitting elements 26 and TFTs 18, as an example of transistors, are illustrated. A substrate 11 made of glass, silicon, or the like and an insulating layer 12 over the substrate are provided. Active elements 18, such as TFTs, are arranged on the insulating layer. In each active element, a gate electrode 13, a gate insulating film 14, and a semiconductor layer 15 are disposed. The TFT 18 further includes a semiconductor layer 15, a drain electrode 16, and a source electrode 17. An insulating film 19 is provided over the TFTs 18. The source electrode 17 is connected to the anode 21 of the corresponding organic light-emitting element 26 through a contact hole 20 formed in the insulating film.

The electrical coupling between the electrode (anode or cathode) of the organic light-emitting element 26 and the electrode (source electrode or drain electrode) of the TFT is not limited to the manner illustrated in Fig. 1B. In other words, either the anode or the cathode is electrically coupled to either the source electrode or drain electrode of the TFT. A TFT refers to a thin film transistor.

Although the display device 100 depicted in Fig. 1B is illustrated as if it had only one organic compound layer, the organic compound layer 22 may have a plurality of layers. A first protective layer 24 and a second protective layer 25 are disposed over the cathodes 23 to reduce the deterioration of the organic light-emitting elements.

Although the display device 100 depicted in Fig. 1B uses transistors as switching elements, other switching elements may be used instead of transistors.

The transistors of the display device 100 depicted in Fig. 1B are not limited to the transistors using a monocrystalline silicon wafer and may be thin film transistors including an active layer on the insulating surface of a substrate. The active layer may be made of monocrystalline silicon, non-monocrystalline silicon, such as amorphous silicon or microcrystalline silicon, or a non-monocrystalline oxide semiconductor, such as indium zinc oxide or indium gallium zinc oxide. Thin film transistors are also referred to as TFT elements.

The transistors in the display device 100 depicted in Fig. 1B may be formed within a substrate such as a Si substrate. To be formed in the substrate implies that the transistors are formed by working the substrate. In other words, transistors formed in a substrate implies that the substrate and the transistors are formed in one body.

In the organic light-emitting element according to an embodiment of the present invention, the emission luminance is controlled by a TFT, which is an example of switching elements, and a plurality of such organic light-emitting elements are arranged in a plane so that respectively controlled emission luminances represent images to be displayed. In the implementation of the present invention, the switching element is not limited to a TFT and may be a transistor made of a low-temperature polysilicon or an active matrix driver on a substrate such as a Si substrate. The phrase "on a substrate" may also mean within the substrate. Whether providing transistors within a substrate or using TFTs depends on the size of the display section. For example, in the case of about 0.5 inch in size, providing organic light-emitting elements on a silicon substrate is preferred.

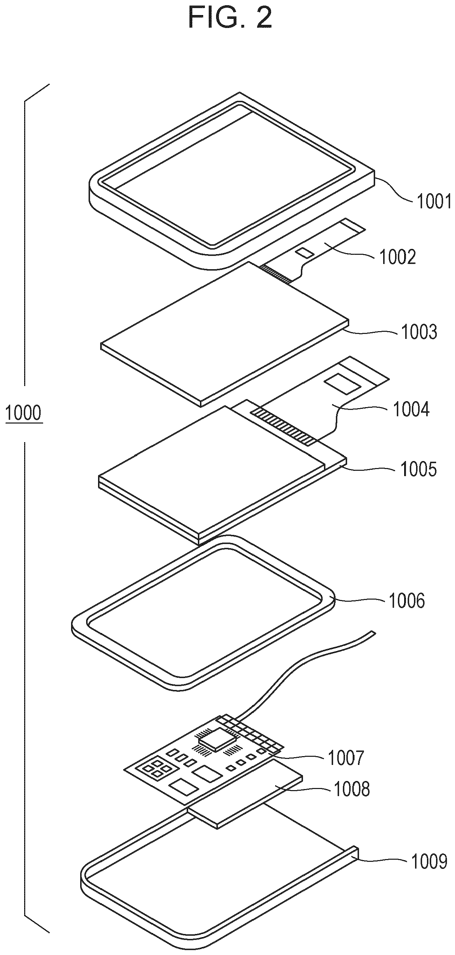

Fig. 2 is a schematic illustrative representation of a display device according to an embodiment of the present invention. The display device 1000 may include a touch panel 1003, a display panel 1005, a frame 1006, a circuit board 1007, and a battery 1008 between an upper cover 1001 and a lower cover 1009. The display panel 1005 may include the organic light-emitting element according to an embodiment of the present invention. The touch panel 1003 and the display panel 1005 are connected to flexible printed circuits, FPCs, 1002 and 1004, respectively. Transistors are printed on the circuit board 1007. The battery 1008 is not necessarily provided unless the display device is for mobile use, and, for mobile use, the battery may be located in another position.

In an embodiment, the display device may include red, green, and blue color filters. The red, green, and blue color filters may be arranged in a delta array.

In an embodiment, the display device may be used in the display section of a mobile terminal. In this instance, the display device may have both a displaying function and an operational function. The mobile terminal may be a cellular phone, such as a smartphone, a tablet PC, or a head-mounted display.

In an embodiment, the display device may be used as the display section of an imaging device including an imaging element that receives light. The display device may have a display section in which information obtained by the imaging element is displayed. The display section may be exposed to the outside of the imaging device or may be disposed within a finder. The imaging device may be a digital camera or a digital video camera.

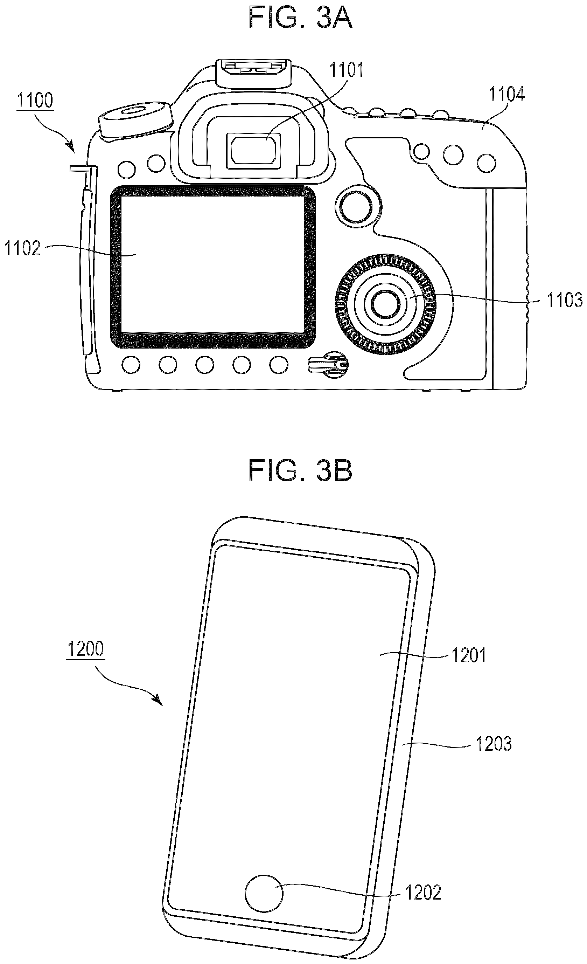

Fig. 3A is a schematic illustrative representation of an imaging device according to an embodiment of the present invention. The imaging device 1100 may include a viewfinder 1101, a rear display 1102, an operational section 1103, and a housing 1104. The viewfinder 1101 and the rear display 1102 may include the organic light-emitting element according to an embodiment of the present invention. In this instance, the viewfinder 1101 and the rear display 1102 may display not only images to be taken but also environmental information, imaging instructions, or the like. The environmental information may include the intensity and the direction of external light, the speed at which the subject moves, and the possibility that an object hides the subject.

Since the appropriate timing for taking an image is only a short period, it is desirable to display information as quickly as possible. Accordingly, a display device that includes the organic light-emitting elements according to an embodiment of the present invention is preferably used. This is because the organic light-emitting element respond quickly. The display device using organic light-emitting elements is more suitably used than liquid crystal display devices in apparatuses required to display information quickly.

The imaging device 1100 may include an optical section, which is not shown in the figure. The optical section includes a single or multiple lenses, which form an image on the imaging element in the housing 1104. In the case of multiple lenses, the focus can be adjusted by adjusting the relative positions of the lenses. This may be automatically performed. The imaging device may be called a photoelectric conversion device. The photoelectric conversion device may take images by a method of detecting differences from the previous image or cutting an image out of images continuously recorded instead of sequentially taking images.

Fig. 3B is a schematic illustrative representation of an electronic apparatus according to an embodiment of the present invention. The electronic apparatus 1200 includes a display section 1201, an operational section 1202, and a housing 1203. The housing 1203 may contain a circuit, a printed board having the circuit, a battery, and a communication section. The operational section 1202 may be a button or a touch panel responder. The operational section may have a biometrically recognizing section that recognizes fingerprints to release a lock. An electronic apparatus including a communication section may be referred to as a communication apparatus. The electronic apparatus may further include a lens and an imaging element to function as a camera. Images taken by the function as a camera are displayed on the display section. Such electronic apparatuses include smartphones and laptop PCs.

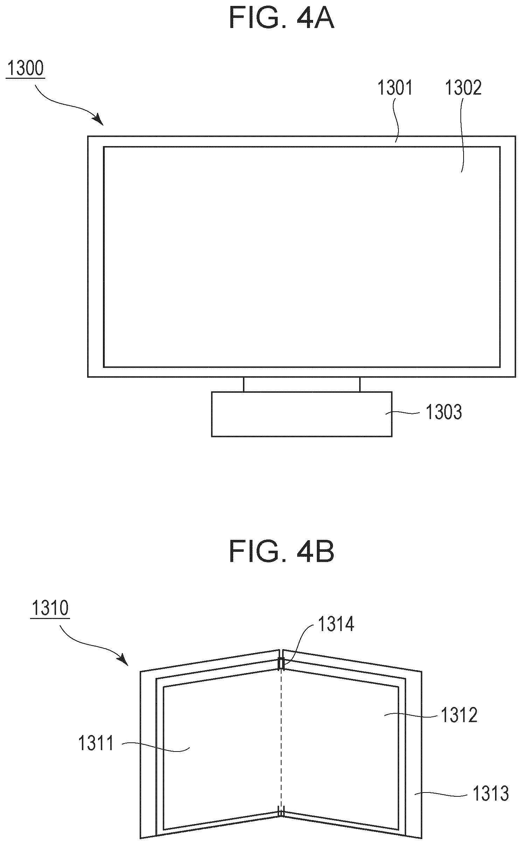

Figs. 4A and 4B are each a schematic illustrative representation of a display device according to an embodiment of the present invention. Fig. 4A illustrates a display device such as a TV monitor or a PC monitor. The display device 1300 includes a housing 1301 and a display section 1302. The display section 1302 may include the organic light-emitting element according to an embodiment of the present invention.

The display device 1300 includes the housing 1301 and a base 1303 supporting the display section 1302. The base 1303 is not limited to the form illustrated in Fig. 4A. The lower side of the housing 1301 may serve as the base.

The housing 1301 and the display section 1302 may be curved. The radius of curvature may be 5000 mm to 6000 mm.

Fig. 4B is a schematic illustrative representation of a display device according to another embodiment of the present invention. The display device 1310 depicted in Fig. 4B is foldable and is, thus, what is called a foldable display device. The display device 1310 includes a first display section 1311, a second display section 1312, and a housing 1313 and has folding points 1314. The first display section 1311 and the second display section 1312 each may include the organic light-emitting element according to an embodiment of the present invention. The first display section 1311 and the second display section 1312 may constitute a seamless one-piece display device. The first display section 1311 and the second display section 1312 can be separated from each other at the folding points. The first display section 1311 and the second display section 1312 may display different images from each other, or a single image may be displayed on a set of the first and second display sections.

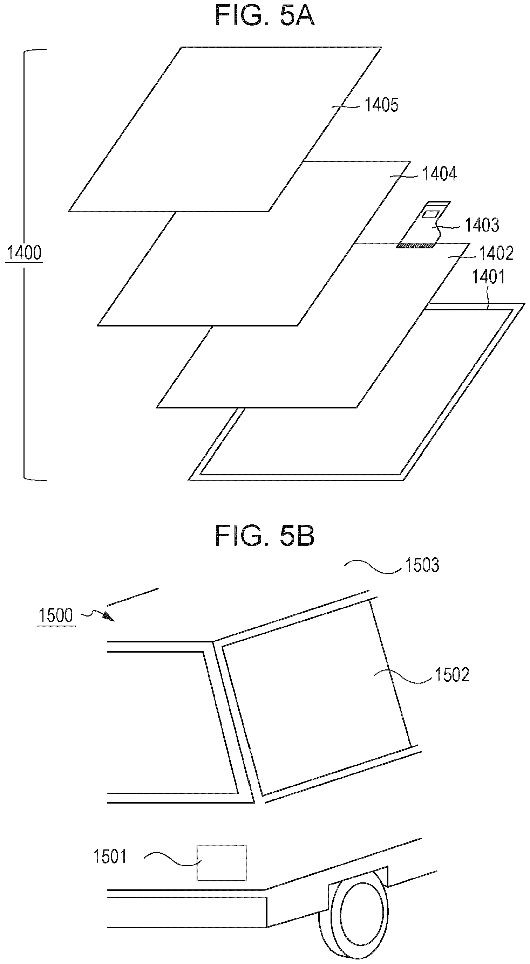

Fig. 5A is a schematic illustrative representation of a lighting device according to an embodiment of the present invention. The lighting device 1400 may include a housing 1401, a light source 1402, a circuit board 1403. The light source 1402 may include the organic light-emitting element according to an embodiment of the present invention. The lighting device 1400 may include an optical film 1404 to improve the color rendering properties of the light source. The lighting device 1400 may also include a light diffusing section 1405 to diffuse light emitted from the light source effectively. The lighting device 1400 including the light diffusing section 1405 enables light to be delivered over a wide area. The optical film 1404 and the light diffusing section 1405 may be disposed on the light emitting side of lighting. A cover may be provided at an outermost portion, if necessary.

The lighting device illuminates, for example, a room. The lighting device may emit light of white, neutral white, or any other color from blue to red. The lighting device may include a dimmer circuit that dims the light. The lighting device may include a power supply circuit. The power supply circuit may be a circuit that converts alternating voltage to direct voltage. White has a color temperature of 4200 K and neutral white has a color temperature of 5000 K. The lighting device may include a color filter.

The lighting device according to an embodiment of the present invention may include a heat radiation section. The heat radiation section is intended to dissipate heat from the device to the outside and may be made of, for example, a metal having a high thermal conductivity or ceramic.

Fig. 5B is a schematic illustrative representation of an automobile that is an implementation of the movable apparatus according to an embodiment of the present invention. The automobile has a tail lamp that is an example of lighting fixtures. The automobile 1500 has a tail lamp 1501, and the tail lamp may be such that it turns on when braking operation or the like is performed. The automobile 1500 may include a car body 1503 and a window 1502 attached to the car body.

The tail lamp 1501 may include the organic light-emitting element according to an embodiment of the present invention. The tail lamp may include a protective member that protects the light source. The protective member may be made of any material provided that it has a certain degree of high strength and is transparent but is preferably made of polycarbonate or the like. The polycarbonate may be mixed with a furandicarboxylic acid derivative, an acrylonitrile derivative, or the like.

In an embodiment of the present invention, the movable apparatus may be a car, a ship, an aircraft, a drone, or the like. The movable apparatus may include an apparatus body and a lighting fixture provided at the apparatus body. The lighting fixture may emit light to notify the position of the apparatus body.

The electronic apparatus or the display device may be applied to systems that can be worn as a wearable device, such as smart glasses, a head-mounted display, or smart contact lenses. The electronic apparatus may include an imaging device capable of photoelectrically converting visible light and a display device capable of emitting visible light.

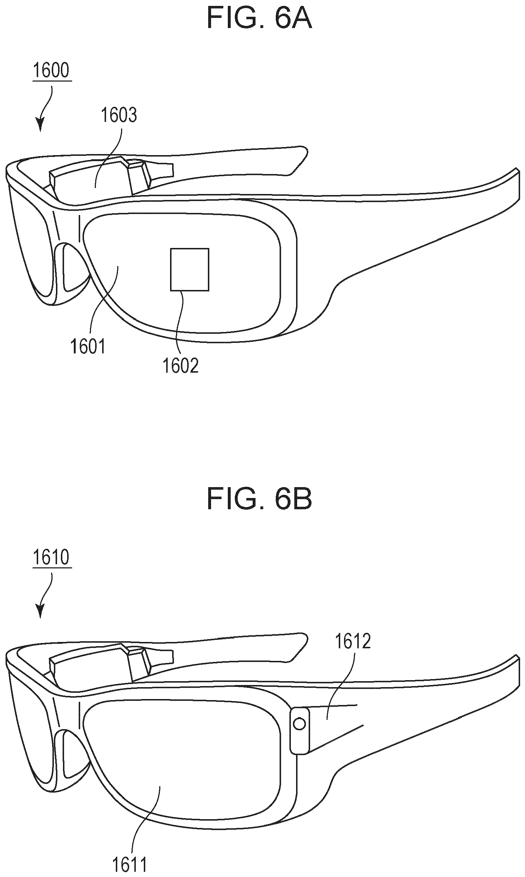

Figs. 6A and 6B are each a schematic illustrative representation of glasses (smart glasses) according to an embodiment of the present invention. The glasses 1600 (smart glasses) will now be described with reference to Fig. 6A. The glasses 1600 include a display section on the rear surface of a lens 1601. The display section may include an organic light-emitting element according to the present invention. The lens 1601 may further be provided with an imaging device 1602, such as a CMOS sensor or a SPAD, on the front surface side.

The glasses 1600 further include a controller 1603. The controller 1603 functions as a power supply to supply power to the imaging device 1602 and the display section. The controller 1603 also controls the operations of the imaging device 1602 and the display section. In the lens 1601, an optical system is formed to focus light on the imaging device 1602 or the display section.

Glasses 1610 (smart glasses) will now be described with reference to Fig. 6B. The glasses 1610 include a controller 1612, and the controller 1612 is provided with a display section including an organic light-emitting element according to the present invention. The controller 1612 may further include an imaging device equivalent to the imaging device 1602. The lens 1611 has an optical system to project emission from the controller 1612, and images are projected on the lens 1611. The controller 1612 functions as a power supply to supply power to the imaging device and the display device and also controls the operations of the imaging device and the display device. The controller may have a sight line detector that detects the wearer's line of sight. Infrared light may be used to detect the line of sight. An infrared emission section emits infrared rays to an eyeball of the user gazing at the displayed image. An imaging section with a light receiving element detects the reflection of emitted infrared light from the eyeball, thus obtaining an image captured by the eyeball. The deterioration of image quality is reduced by providing a reducing means that reduces the light in plan view to the display section from the infrared emission section.

The controller 1612 detects the user's line of sight to the displayed image from the captured image of the eyeball obtained by capturing the infrared light. Known techniques can be applied to detect the line of sight using a captured image of the eyeball. For example, a sight line detection method based on the Purkinje image formed by the reflection of irradiation light at the cornea can be used.

More specifically, sight line detection is performed based on pupil center corneal reflection. The sight line vector representing the direction (rotation angle) of the eyeball is calculated based on the image of the pupil and the Purkinje image included in the captured image of the eyeball, using pupil center corneal reflection, thus detecting the user's line of sight.

In an embodiment of the present invention, the display device may include an imaging device having a light receiving element, and the image displayed on the display device may be controlled based on the user's line-of-sight information from the imaging device.

More specifically, the display device determines a first view field area at which the user gazes and a second view field area other than the first view field area based on the line-of-sight information. The first view field area and the second view field area may be determined by the controller of the display device, or the display device may receive those determined by an external controller. In the display region of the display device, the display resolution of the first view field area may be controlled to be higher than that of the second view field area. In other words, the resolution of the second view field area may be lower than that of the first view field area.

A first display area or a display area with higher priority may be determined using AI. The AI may be a model configured to estimate the angle of the line of sight from the image of the eyeball and the distance to an object beyond the line of sight, using as teacher data the image of the eyeball and the direction of the actual eyeball look. The AI may be included in the display device, the imaging device, or an external device. When an external device includes the AI, smart glasses further including an imaging device that takes external images are preferred. The smart glasses can display information about external images taken in real time.

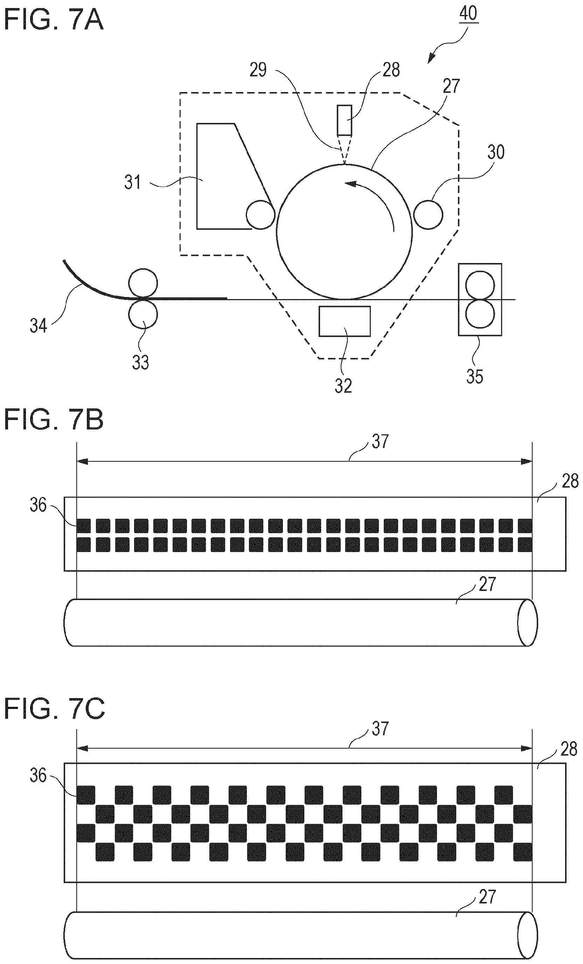

Fig. 7A is a schematic illustrative representation of an image forming apparatus according to an embodiment of the present invention. The image forming apparatus 40, which is an electrophotographic image forming apparatus, includes a photosensitive member 27, an exposure light source 28, a charging section 30, a developing section 31, a transfer device 32, conveying rollers 33, and a fuser 35. The exposure light source 28 emits light 29 to form an electrostatic latent image on the surface of the photosensitive member 27. The exposure light source 28 may include the organic light-emitting element according to an embodiment of the present invention. The developing section 31 contains a toner or the like. The charging section 30 charges the photosensitive member 27. The transfer device 32 transfers developed images to a storage medium 34. The conveying rollers 33 convey the recording medium 34. The recording medium 34 is, for example, a paper sheet. The fuser 35 fixes the image formed on the recording medium 34.

Figs. 7B and 7C are each a schematic illustrative representation of the exposure light sources 28 having emitting portions 36 arranged on a long substrate. Arrows 37 each indicate the row direction in which organic light-emitting elements are arranged. The row direction is the same as the direction of the rotation axis of the photosensitive member 27. This direction can be called the longitudinal axis direction of the photosensitive member 27. Fig. 7B illustrates a form in which the emitting portions 36 are arranged along the longitudinal direction of the photosensitive member 27. Fig. 7C illustrates a form different from the form in Fig. 7B, in which the emitting portions 36 are arranged alternately in first rows and second rows in the row direction. The emitting portions in the first rows and those in the second rows are at different positions in the column direction. In the first rows, the emitting portions 36 are aligned at intervals. In the second rows, the emitting portions 36 are placed at positions corresponding to the spaces between the emitting portions 36 in the first rows. Thus, the emitting portions 36 are arranged at intervals also in the column direction. The arrangement in Fig. 7C can be rephrased as, for example, a lattice arrangement, a staggered arrangement, or a checkerboard pattern.

As described above, the device including the organic light-emitting elements according to an embodiment of the present invention can display high-quality images stably in long-time displaying.

### EXAMPLES

The present invention will be further described with reference to Examples below. It should be appreciated that the present invention is not limited to the following Examples.

### [EXAMPLE 1 (Synthesis of Exemplified Compound A1)]

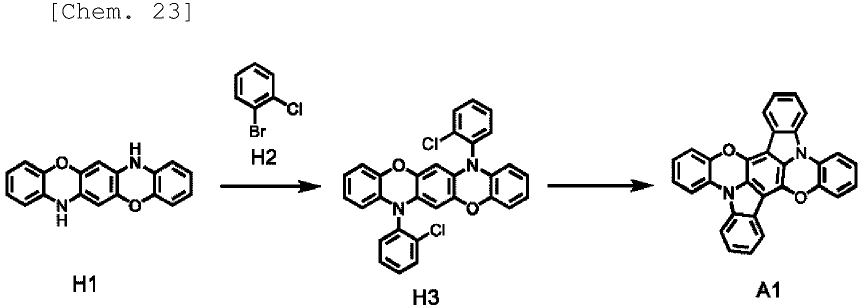

### (1) Synthesis of Compound H3

The following reagents and solvents presented below were placed into a 500 mL recovery flask.
Compound H1: 500 mg (1.73 mmol)
Compound H2: 1.33 g (6.94 mmol)
Pd(dba)₂: 45 mg (0.09 mmol)
DPPF: 143 mg (0.27 mmol)
t-BuONa: 664 mg (6.92 mmol)
Toluene: 200 mL

Subsequently, the reaction liquid was heated to 110°C under a nitrogen stream and then stirred at this temperature (110°C) for 5 hours. After completion of the reaction, the reaction product was extracted with toluene and water. The extract was concentrated and purified by silica gel column chromatography (toluene) to afford 546 mg (yield: 62%) of Compound H3.

### (2) Synthesis of Exemplified Compound A1

The following reagents and solvents presented below were placed into a 100 mL recovery flask.
Compound H1: 500 mg (0.98 mmol)
Pd(OAc)₂: 6 mg (0.03 mmol)
P(t-Bu)3: 20 mg (0.09 mmol)
DBU: 596 mg (3.92 mmol)
o-Xylene: 30 mL

Subsequently, the reaction liquid was heated to 140°C under a nitrogen stream and then stirred at this temperature (140°C) for 6 hours. After completion of the reaction, methanol was added, and the product was filtered and rinsed with methanol. The resulting product was purified by silica gel column chromatography (chlorobenzene) to afford 47 mg (yield: 110) of Exemplified Compound A1.

Exemplified compound A1 was subjected to mass spectrometry using MALDI-TOF-MS (Autoflex LRF manufactured by Bruker).

### [MALDI-TOF-MS]

Measured value: m/z = 436, Calculation value: C₄₂H₂₄B₂N₄ = 436

### [EXAMPLES 2 to 31 (Synthesis of Exemplified Compounds)]

Exemplified compounds of Examples 2 to 31 were synthesized in the same manner as in Example 1, except for replacing raw materials H1 and H2 used in Example 1 with raw materials 1 and 2, respectively. The raw materials 1 and 2 used in the Examples are presented in Tables 4-1 to 4-6.

The measured values m/z of the mass spectrometry results obtained in the same manner as in Example 1 are also presented.

### [Table 4-1]

**Table 4-1**

| Example | Exemplified compound | Raw material 1 | Raw material 2 | m/z |
|---|---|---|---|---|
| 2 | A2 | 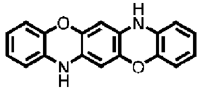 | 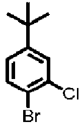 | 549 |
| 3 | A3 | 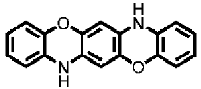 | 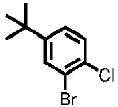 | 549 |
| 4 | A4 | 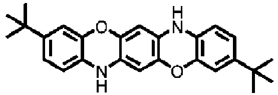 | 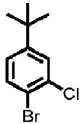 | 661 |
| 5 | A5 | 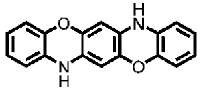 | 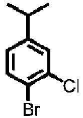 | 521 |
| 6 | A8 | 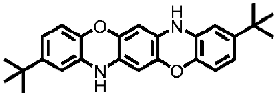 | 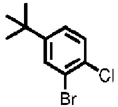 | 661 |
| 7 | A9 | 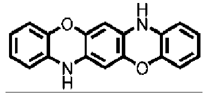 | 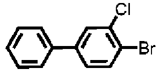 | 589 |
| 8 | A11 | 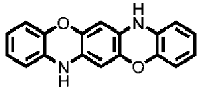 | 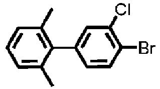 | 645 |
| 9 | A15 | 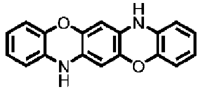 | 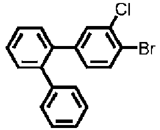 | 741 |

### [Table 4-2]

**Table 4-2**

| Example | Exemplified compound | Raw material 1 | Raw material 2 | m/z |
|---|---|---|---|---|
| 10 | A17 | 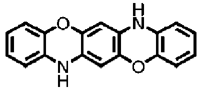 | 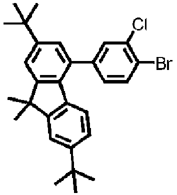 | 1045 |
| 11 | A22 | 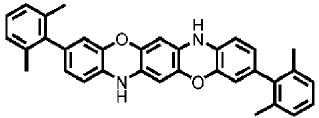 | 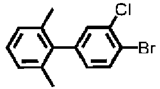 | 853 |
| 12 | A24 | 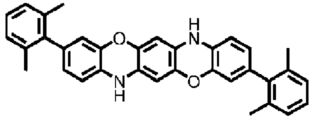 | 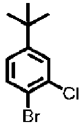 | 757 |
| 13 | B1 | 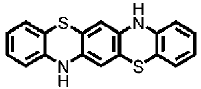 | 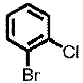 | 469 |
| 14 | B2 | 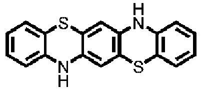 | 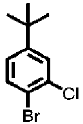 | 581 |
| 15 | B11 | 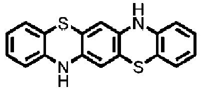 | 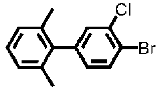 | 677 |
| 16 | B25 | 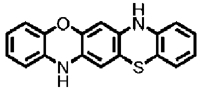 | 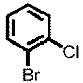 | 453 |
| 17 | B37 | 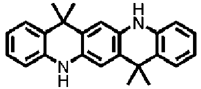 | 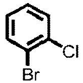 | 489 |

### [Table 4-3]

**Table 4-3**

| Example | Exemplified compound | Raw material 1 | Raw material 2 | m/z |
|---|---|---|---|---|
| 18 | B38 | 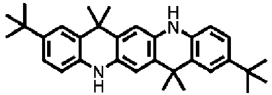 | 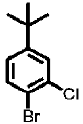 | 713 |
| 19 | B39 | 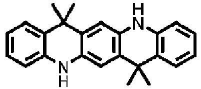 | 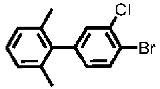 | 697 |
| 20 | C1 | 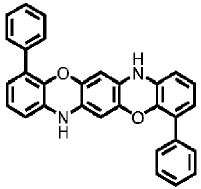 | 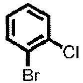 | 589 |
| 21 | C2 | 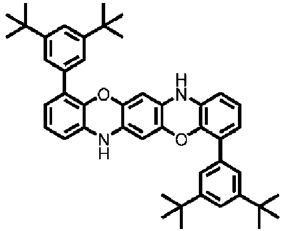 | 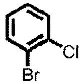 | 813 |
| 22 | C6 | 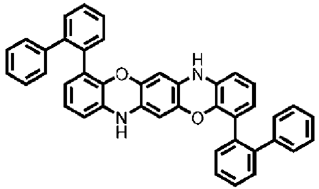 | 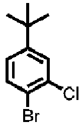 | 853 |
| 23 | C9 | 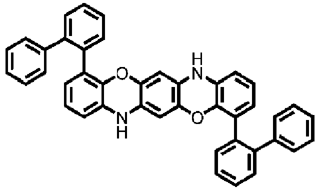 | 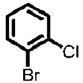 | 741 |

### [Table 4-4]

**Table 4-4**

| Example | Exemplified compound | Raw material 1 | Raw material 2 | m/z |
|---|---|---|---|---|
| 18 | B38 | 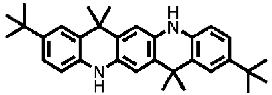 | 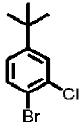 | 713 |
| 19 | B39 | 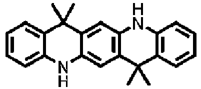 | 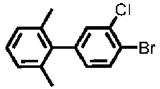 | 697 |
| 20 | C1 | 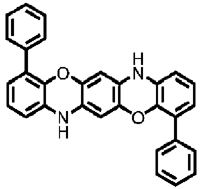 | 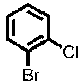 | 589 |
| 21 | C2 | 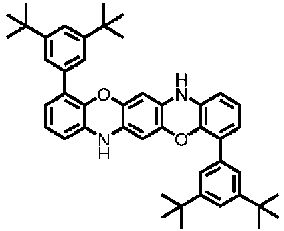 | 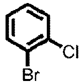 | 813 |
| 22 | C6 | 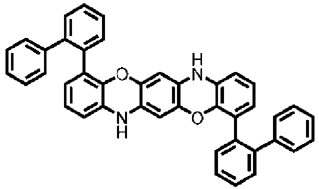 | 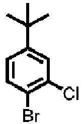 | 853 |

### [Table 4-5]

**Table 4-5**

| Example | Exemplified compound | Raw material 1 | Raw material 2 | m/z |
|---|---|---|---|---|
| 23 | C9 | 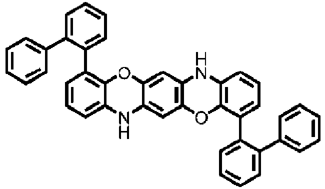 | 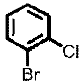 | 741 |
| 24 | C17 | 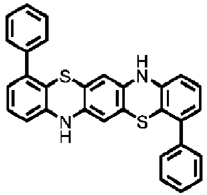 | 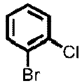 | 621 |
| 25 | D1 | 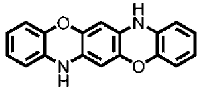 | 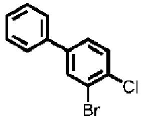 | 589 |
| 26 | D4 | 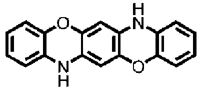 | 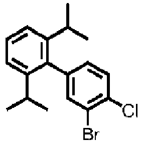 | 757 |
| 27 | D8 | 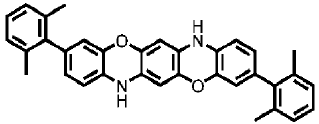 | 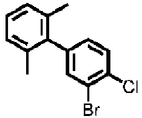 | 853 |
| 28 | E1 | 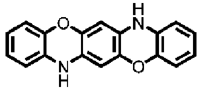 | 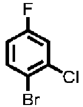 | 472 |
| 29 | E8 | 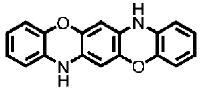 | 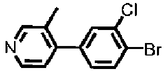 | 619 |

### [Table 4-6]

**Table 4-6**

| Example | Exemplified compound | Raw material 1 | Raw material 2 | m/z |
|---|---|---|---|---|
| 30 | E10 | 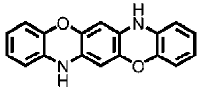 | 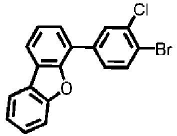 | 769 |
| 31 | E13 | 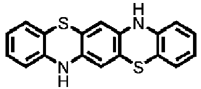 | 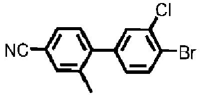 | 699 |

### [EXAMPLE 32]

In the present Example, an organic EL element having a bottom emission structure was produced which included an anode, a hole injection layer, a hole transport layer, an electron blocking layer, a luminescent layer, a hole blocking layer, an electron transport layer, an electron injection layer, and a cathode, in this order, on a substrate.

First, ITO was deposited on a glass substrate and then subjected to desired patterning to form an ITO electrode (anode). The thickness of the ITO electrode was 100 nm at this time. The resulting substrate with the ITO electrode was used as an ITO substrate in the following step. Then, organic compound layers and an electrode layer, as presented in Table 5, were continuously formed on the ITO substrate by vapor deposition with resistance heating in a vacuum chamber. In this operation, the area of the opposing electrode (metal electrode layer, cathode) was adjusted to 3 mm².

### [Table 5]

**Table 5**

| | Material | | | Thickness (nm) |
|---|---|---|---|---|
| Cathode | Al | | | 100 |
| Electron injection layer (EIL) | LiF | | | 1 |
| Electron transport layer (ETL) | ET2 | | | 15 |
| Hole blocking layer (HBL) | ET12 | | | 15 |
| Luminescent layer (EML) | Host | EM4 | Mass ratio EM4:A2 =98:2 | 20 |
| | Guest | A2 | | |
| Electron blocking layer (EBL) | HT12 | | | 15 |
| Hole transport layer (HTL) | HT3 | | | 30 |
| Hole injection layer (HIL) | HT16 | | | 5 |

The properties of the resulting element were measured and evaluated. It was confirmed that blue emission was obtained from the emission spectrum of the light-emitting element at a luminance of 1000 cd/m². The current-voltage characteristics were measured with a microammeter 4140B manufactured by Hewlett Packard, and the emission luminance was measured with BM7 manufactured by Topcon. Furthermore, a continuous driving test was conducted at a current density of 20 mA/cm², and the period (LT95) until when the luminance was 5% degraded from the initial luminance was measured. The result was 130 hours.

### [EXAMPLES 33 to 51, COMPARATIVE EXAMPLE 1]

Organic light-emitting elements were prepared in the same manner as in Example 32, except that the compounds were replaced with the compounds presented in Table 6 as needed. The properties of the resulting element were measured and evaluated in the same manner as in Example 32. The measurement results are presented in Table 6.

### [Table 6]

**Table 6**

| | HIL | HTL | EBL | EML | | HBL | ETL | LT95 [h] | Emission color | Half-width |
|---|---|---|---|---|---|---|---|---|---|---|
| | | | | Host | Luminescent material | | | | | |
| Example 33 | HT16 | HT3 | HT12 | EM27 | A4 | ET10 | ET2 | 135 | Blue | AA |
| Example 34 | HT16 | HT3 | HT9 | EM5 | A4 | ET12 | ET2 | 125 | Blue | AA |
| Example 35 | HT16 | HT3 | HT12 | EM26 | A9 | ET12 | ET2 | 139 | Blue | A |
| Example 36 | HT16 | HT3 | HT12 | EM4 | A11 | ET12 | ET2 | 134 | Blue | A |
| Example 37 | HT16 | HT3 | HT12 | EM4 | A17 | ET12 | ET2 | 132 | Blue | A |
| Example 38 | HT16 | HT3 | HT12 | EM27 | A22 | ET12 | ET2 | 137 | Blue | A |
| Example 39 | HT16 | HT3 | HT12 | EM4 | A24 | ET12 | ET2 | 132 | Blue | A |
| Example 40 | HT16 | HT3 | HT12 | EM27 | B2 | ET12 | ET2 | 132 | Blue | A |
| Example 41 | HT16 | HT3 | HT12 | EM26 | B11 | ET12 | ET5 | 134 | Blue | A |
| Example 42 | HT16 | HT3 | HT9 | EM26 | B38 | ET12 | ET5 | 125 | Blue | A |
| Example 43 | HT16 | HT3 | HT12 | EM12 | C1 | ET12 | ET2 | 137 | Blue | A |
| Example 44 | HT16 | HT3 | HT12 | EM4 | C2 | ET12 | ET2 | 134 | Blue | A |
| Example 45 | HT16 | HT2 | HT12 | EM26 | C9 | ET10 | ET2 | 136 | Blue | A |
| Example 46 | HT16 | HT3 | HT12 | EM5 | C17 | ET12 | ET2 | 125 | Blue | A |
| Example 47 | HT16 | HT3 | HT12 | EM5 | D4 | ET12 | ET2 | 129 | Blue | A |
| Example 48 | HT16 | HT3 | HT9 | EM5 | E1 | ET10 | ET2 | 125 | Blue | A |
| Example 49 | HT16 | HT3 | HT12 | EM5 | E8 | ET12 | ET5 | 123 | Blue | A |
| Example 50 | HT16 | HT3 | HT12 | EM27 | E10 | ET12 | ET2 | 138 | Blue | A |
| Example 51 | HT16 | HT3 | HT9 | EM5 | E13 | ET12 | ET5 | 124 | Blue | A |
| Comparative Example 1 | HT16 | HT3 | HT12 | EM4 | Comparative Compound 1-a | ET12 | ET2 | 75 | Light blue | C |

As shown in Table 6, the elements using the organic compound of the present invention exhibited good durability. This is because the compounds according to the present invention can emit blue light with a narrow half-width and excellent color purity because of the presence of the skeleton in which at least one of the substituents at the two nitrogen atoms in the main skeleton is fused to the main skeleton. Furthermore, these compounds exhibited excellent durability because the fused ring structure includes a C-C bond with high bonding energy and is accordingly stable in terms of molecular structure. In contrast, in Comparative Example 1 using Comparative Compound 1-a, which is described in PTL 1, the emission color was light blue and had a large half-width. The LT95 of Comparative Compound 1-a was 75 hours, and the color purity, current efficiency, and durability are inferior to those of the blue light-emitting elements of the Examples.

### [EXAMPLE 52]

Organic light-emitting elements were prepared in the same manner as in Example 32, except that the compounds were replaced with the compounds presented in Table 7 as needed. The properties of the resulting element were measured and evaluated in the same manner as in Example 32.

### [Table 7]

**Table 7**

| | Material | | | Thickness (nm) |
|---|---|---|---|---|
| Cathode | Al | | | 100 |
| Electron injection layer (EIL) | LiF | | | 1 |
| Electron transport layer (ETL) | ET2 | | | 10 |
| Hole blocking layer (HBL) | ET12 | | | 20 |
| Luminescent layer (EML) | First host | EM11 | Mass ratio EM3:A1:GD1 =89.7:10.0:0.3 | 30 |
| | Second host | A1 | | |
| | Guest | GD1 | | |
| Electron blocking layer (EBL) | HT12 | | | 15 |
| Hole transport layer (HTL) | HT3 | | | 30 |
| Hole injection layer (HIL) | HT16 | | | 5 |

The properties of the resulting element were measured and evaluated. The measurement results are presented in Table 8. These results suggest that the light-emitting element produced good green emission. The current-voltage characteristics were measured with a microammeter 4140B manufactured by Hewlett Packard, and the emission luminance was measured with BM7 manufactured by Topcon. Furthermore, a continuous driving test was conducted at a current density of 10 mA/cm², and the LT95 was measured. The result was over 500 hours.

### [EXAMPLES 53 to 64, COMPARATIVE EXAMPLE 2]

Organic light-emitting elements were prepared in the same manner as in Example 54, except that the compounds were replaced with the compounds presented in Table 8 as needed. The properties of the resulting elements were measured and evaluated in the same manner as in Example 54. The measurement results are presented in Table 8.

### [Table 8]

**Table 8**

| | EML | | | Emission color | LT95 [h] |
|---|---|---|---|---|---|
| | First host | Second host | Guest | | |
| Example 53 | EM11 | A1 | GD1 | Green | 550 |
| Example 54 | EM13 | A9 | GD1 | Green | 560 |
| Example 55 | EM11 | B37 | GD1 | Green | 510 |
| Example 56 | EM13 | E10 | GD1 | Green | 530 |
| Example 57 | EM3 | A1 | RD1 | Red | 700 |
| Example 58 | EM9 | A9 | RD1 | Red | 710 |
| Example 59 | EM3 | B37 | RD1 | Red | 650 |
| Example 60 | EM9 | E10 | RD1 | Red | 680 |
| Example 61 | A9 | | GD1 | Green | 530 |
| Example 62 | E10 | | GD1 | Green | 510 |
| Example 63 | A9 | | RD1 | Red | 660 |
| Example 64 | E10 | | RD1 | Red | 640 |
| Comparative Example 2 | EM11 | Comparative Compound 1-a | GD1 | Green | 300 |

Table 8 shows that the elements using any of the organic compounds according to the present invention exhibit LT95 exceeding 500 hours and are thus excellent in durability. In contrast, the organic light-emitting element of Comparative Example 2, whose LT95 is less than 500 hours, is not good in durability.

### [EXAMPLE 65]

In the present Example, an organic EL element having a top emission structure was produced which includes an anode, a hole injection layer, a hole transport layer, an electron blocking layer, a first luminescent layer, a second luminescent layer, a hole blocking layer, an electron transport layer, an electron injection layer, and a cathode, in this order, on a substrate.

Al and Ti were deposited to form a 40 nm-thick multilayer film on a glass substrate by sputtering, and the multilayer film was patterned by photolithography to form the anode. In this operation, the area of the opposing electrode (metal electrode layer, cathode) was adjusted to 3 mm². Subsequently, the cleaned substrate on which up to the electrode were formed and materials were set in a vapor deposition apparatus (manufactured by ULVAC), which was then evacuated to 1.3 × 10⁻⁴ Pa (1 × 10⁻⁶ Torr), and were subjected to UV/ozone cleaning. Then, the layers in the layered structure presented in Table 9 were formed, finally sealing the resulting structure in a nitrogen atmosphere.

### [Table 9]

**Table 9**

| | Material | | | Thickness (nm) |
|---|---|---|---|---|
| Cathode | Mg | | Mass ratio Mg:Ag = 50:50 | 10 |
| | Ag | | | |
| Electron injection layer (EIL) | LiF | | | 1 |
| Electron transport layer (ETL) | ET2 | | | 25 |
| Hole blocking layer (HBL) | ET12 | | | 80 |
| Second luminescent layer (2nd EML) | Second host | EM1 | Mass ratio EM1:A2 =98:2 | 15 |
| | Second guest (Blue dopant) | A2 | | |
| First luminescent layer (1st EML) | First host | EM1 | Mass ratio EM1:RD1:GD7 = 96.7:0.3:3.0 | 10 |
| | First guest (Red dopant) | RD1 | | |
| | Third guest (Green dopant) | GD7 | | |
| Electron blocking layer (EBL) | HT12 | | | 10 |
| Hole transport layer (HTL) | HT3 | | | 20 |
| Hole injection layer (HIL) | HT16 | | | 5 |

The properties of the resulting element were measured and evaluated. The resulting element produced white emission with excellent color purity. The element was further subjected to a continuous driving test at an initial luminance of 1000 cd/m², and the degradation in luminance after 100 hours was measured. As a result, the luminance was 26% degraded.

### [EXAMPLES 66 to 68, COMPARATIVE EXAMPLE 3]

Organic light-emitting elements were prepared in the same manner as in Example 66, except that the compounds were replaced with the compounds presented in Table 10 as needed. The properties of the resulting elements were measured and evaluated in the same manner as in Example 66. The measurement results are presented in Table 10.

### [Table 10]

**Table 10**

| | 1^{st} EML | | | 2^{nd} EML | | Luminance degradation [%] |
|---|---|---|---|---|---|---|
| | First host | First guest | Third guest | Second host | Second guest | |
| Example 66 | EM1 | RD1 | GD7 | EM1 | A4 | 27 |
| Example 67 | EM27 | RD1 | GD7 | EM27 | A11 | 23 |
| Example 68 | EM1 | RD1 | GD7 | EM1 | E10 | 25 |
| Comparative Example 3 | EM1 | RD1 | GD7 | EM1 | Comparative Compound 1-a | 52 |

Table 10 suggests that the elements using any of the organic compounds according to the present invention are excellent in durability. This is because the compounds according to the present invention have a highly stable molecular structure because of the structure in which at least one of the substituents at the two nitrogen atoms is fused to the main skeleton and, therefore, less likely to dissociate. In contrast, the luminance of the organic light-emitting element using Comparative Compound 1-a was 52% degraded. This is because when Comparative Compound 1-a is used as the guest, the substituents at the two nitrogen atoms of the main skeleton are not fused and are likely to dissociate, and thus the molecular structure is inferior in stability.

### [EXAMPLE 69]

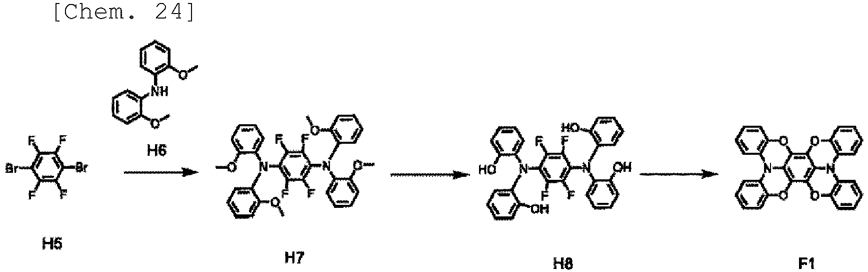

### (1) Synthesis of Compound H7

The following reagents and solvents presented below were placed into a 300 mL recovery flask.
Compound H5: 2.00 g (6.50 mmol)
Compound H6: 2.98 g (13.0 mmol)
Pd(OAc)₂: 69 mg (0.05 mmol)
P(t-Bu)₃: 217 mg (0.09 mmol)
NaOtBu: 2.50 g (26.0 mmol)
o-Xylene: 150 mL

Subsequently, the reaction liquid was heated to 140°C under a nitrogen stream and then stirred at this temperature (140°C) for 6 hours. After completion of the reaction, the organic phase was extracted with toluene. The extract was purified by silica gel column chromatography (toluene, heptane) to afford 943 mg (yield: 24%) of Compound H7.

### (2) Synthesis of Compound H8

The following reagents and solvents presented below were placed into a 50 mL recovery flask.
1-Decanethiol: 389 mg (2.23 mmol)
KOtBu: 250 mg (2.23 mmol)
DMF: 10 mL

Then, these materials were stirred at room temperature for 15 minutes, and to this reaction liquid was added the following compound:
Compound H7: 900 mg (1.49 mmol)

Subsequently, the reaction liquid was heated to 110°C under a nitrogen stream and then stirred at this temperature (110°C) for 3 hours. After completion of the reaction, water was added at room temperature, and 1 M hydrochloric acid was dropped until the pH reached 1. The resulting mixture was subjected to extraction of the organic phase with ethyl acetate. The extract was concentrated and then rinsed with heptane to afford 482 mg (yield: 59%) of Compound H8.

### (3) Synthesis of Exemplified Compound F1

The following reagents and solvents presented below were placed into a 100 mL recovery flask.
Compound H8: 450 mg (0.82 mmol)
K₂CO₃: 735 mg (6.56 mmol)
DMAc: 20 mL

Subsequently, the reaction liquid was heated to 120°C under a nitrogen stream and then stirred at this temperature (120°C) for 4 hours. After completion of the reaction, water was added at room temperature and filtered to obtain a crude product. The crude product was heated and dispersed with silica gel in xylene solvent at 120°, followed by Celite filtration. After being concentrated, the filtrate was heated, dispersed, and rinsed using toluene and ethanol (1:1 in volume ratio) to afford 46 mg (yield: 12%) of Exemplified Compound F1.

Exemplified compound F1 was subjected to mass spectrometry using MALDI-TOF-MS (Autoflex LRF manufactured by Bruker).

### [MALDI-TOF-MS]

Measured value: m/z = 468, Calculation value: C₃₀H₁₆N₂O₄ = 468

### [EXAMPLES 70 to 84 (Synthesis of Exemplified Compounds)]

Exemplified compounds of Examples 70 to 84 were synthesized in the same manner as in Example 69, except for replacing raw materials H6 used in Example 69 with raw material 3. Raw material 3 used in the Examples is presented in Tables 11-1 and 11-2.

The measured values m/z of the mass spectrometry results obtained in the same manner as in Example 69 are also presented.

### [Table 11-1]

**Table 11-1**

| Example | Exemplified compound | Raw material 3 | m/z |
|---|---|---|---|
| 70 | F2 | 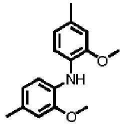 | 525 |
| 71 | F4 | 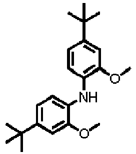 | 693 |
| 72 | F8 | 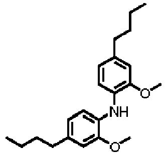 | 693 |
| 73 | F11 | 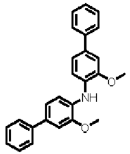 | 773 |
| 74 | F12 | 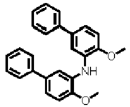 | 773 |
| 75 | F14 | 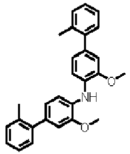 | 829 |
| 76 | F18 | 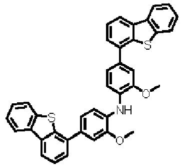 | 1197 |
| 77 | F20 | 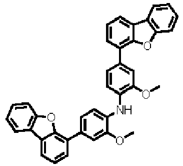 | 1133 |

### [Table 11-2]

**Table 11-2**

| Example | Exemplified compound | Raw material 3 | m/z |
|---|---|---|---|
| 78 | F23 | 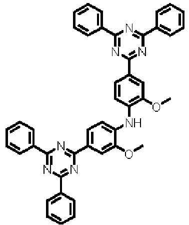 | 1394 |
| 79 | F26 | 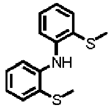 | 533 |
| 80 | F28 | 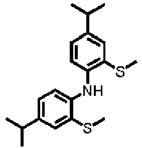 | 701 |
| 81 | F36 | 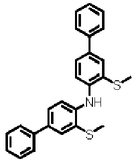 | 837 |
| 82 | F39 | 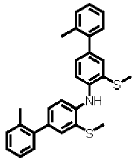 | 893 |
| 83 | F43 | 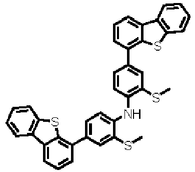 | 1262 |
| 84 | F48 | 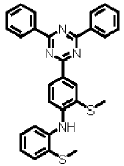 | 995 |

### [EXAMPLES 85 to 95]

Organic light-emitting elements were prepared in the same manner as in Example 32, except that the compounds were replaced with the compounds presented in Table 12 as needed.

The properties of the resulting elements were measured and evaluated. The measurement results are presented in Table 12. The current-voltage characteristics were measured with a microammeter 4140B manufactured by Hewlett Packard, and the emission luminance was measured with BM7 manufactured by Topcon. Furthermore, a continuous driving test was conducted at a current density of 100 mA/cm², and the LT95 was measured. The measurement results are presented in Table 12. In the Table, the percentage in the parentheses represents the concentration of the compound in the luminescent layer.

### [Table 12]

**Table 12**

| | EBL | EML | | | Emission color | LT95 [h] |
|---|---|---|---|---|---|---|
| | | First host | Second host | Guest | | |
| Example 85 | HT12 | EM1 | F1 (15%) | BD7 (1%) | Blue | 20 |
| Example 86 | HT12 | EM5 | F1 (15%) | BD7 (1%) | Blue | 13 |
| Example 87 | HT12 | EM1 | F4 (15%) | BD7 (1%) | Blue | 18 |
| Example 88 | HT12 | EM5 | F4 (15%) | BD7 (1%) | Blue | 12 |
| Example 89 | HT12 | EM1 | F11 (15%) | BD7 (1%) | Blue | 23 |
| Example 90 | HT12 | EM5 | F11 (15%) | BD7 (1%) | Blue | 14 |
| Example 91 | HT12 | EM1 | F1 (15%) | BD4 (1%) | Blue | 17 |
| Example 92 | HT12 | EM5 | F1 (15%) | BD4 (1%) | Blue | 12 |
| Example 93 | F11 | EM1 | F11 (15%) | BD7 (1%) | Blue | 22 |
| Example 94 | HT10 | EM35 | F1 (45%) | GD12 (10%) | Green | 9 |
| Example 95 | F1 | EM35 | F1 (45%) | GD12 (10%) | Green | 11 |

Table 12 shows that the organic light-emitting elements using any of the organic compounds according to the present invention as the second host (assist material) for the blue guest exhibited LT95 of over 10 hours.
Also, the organic light-emitting elements using any of the organic compounds of the present invention as the second host (assist material) for the green guest material exhibited an LT95 of about 10 hours. Furthermore, the organic luminescent elements using any of the organic compounds according to the present invention as EBL exhibited particularly excellent durability among the organic light-emitting elements in Table 12. It was thus found that organic light-emitting elements using any of the organic compounds according to the present invention have excellent durability.

As described above, the organic compounds according to the present invention can emit blue light with excellent color purity and provide excellent durability. In addition, the organic compounds according to the present invention can provide highly durable organic light-emitting elements when used as the host material, assist material, or electron blocking material.

The present invention can have the following constitutions.

### (Constitution 1)

An organic compound represented by general formula (1):

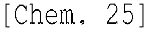

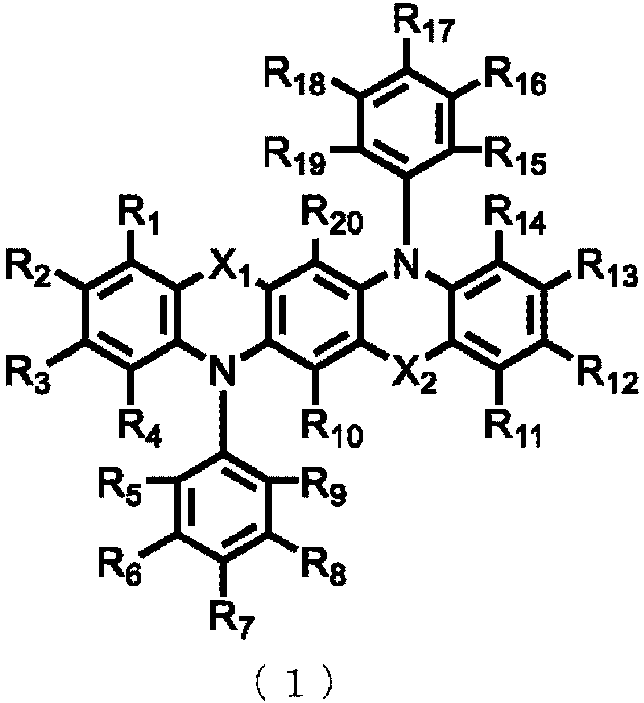

(In general formula (1), R₁ to R₂₀ are each independently selected from the group consisting of a hydrogen atom, a deuterium atom, halogen atoms, substituted or unsubstituted alkyl groups, substituted or unsubstituted alkoxy groups, substituted or unsubstituted silyl groups, substituted or unsubstituted amino groups, substituted or unsubstituted aryl groups, substituted or unsubstituted heteroaryl groups, and a cyano group, wherein R₁ to R₂₀ may be bound to one another to form a ring and, optionally, via a chalcogen atom. At least a combination R₉ and R₁₀ or R₁₉ and R₂₀ forms a bond. X₁ and X₂ are each independently selected from the group consisting of chalcogen atoms, substituted or unsubstituted imino groups, substituted or unsubstituted methylene groups, and substituted or unsubstituted silylene groups.)

### (Constitution 2)

The organic compound of Constitution 1, wherein the organic compound is represented by general formula (2) or (3) :

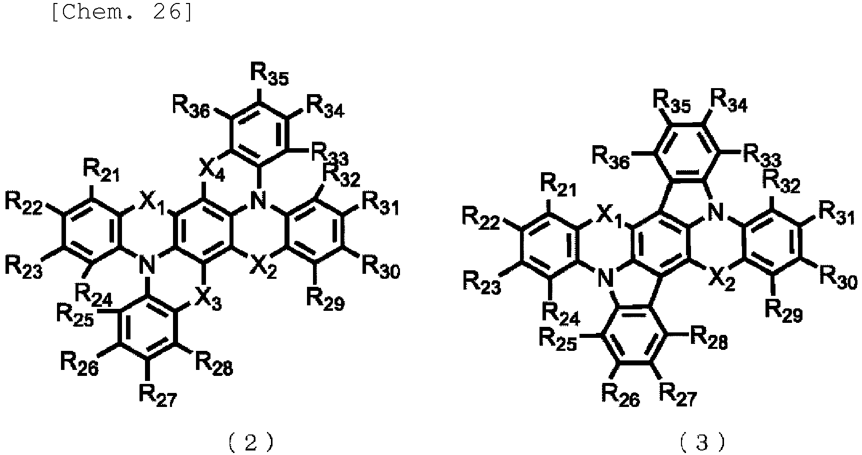

(In general formulas (2) and (3), R₂₁ to R₃₆ are each independently selected from the group consisting of a hydrogen atom, a deuterium atom, halogen atoms, substituted or unsubstituted alkyl groups, substituted or unsubstituted alkoxy groups, substituted or unsubstituted silyl groups, substituted or unsubstituted amino groups, substituted or unsubstituted aryl groups, substituted or unsubstituted heteroaryl groups, and a cyano group. R₂₁ to R₃₆ may be bonded to one another to form a ring and, optionally, via a chalcogen atom. X₁ to X₄ are each independently selected from the group consisting of chalcogen atoms, substituted or unsubstituted imino groups, substituted or unsubstituted methylene groups, and substituted or unsubstituted silylene groups.)

### (Constitution 3)

The organic compound of Constitution 2, wherein in general formulas (2) and (3), at least one of R₂₁, R₂₂, R₂₃, R₂₆, R₂₇, R₂₉, R₃₀, R₃₁, R₃₄, and R₃₅ is other than the hydrogen atom.

### (Constitution 4)

The organic compound of Constitution 2 or 3, wherein in general formulas (2) and (3), at least one of R₂₁, R₂₂, R₂₃, R₂₆, R₂₇, R₂₉, R₃₀, R₃₁, R₃₄, and R₃₅ is an alkyl group with 1 to 10 carbon atoms, an aryl group with 6 to 12 carbon atoms, or a heteroaryl group with 3 to 12 carbon atoms.

### (Constitution 5)

The organic compound of any one of Constitutions 2 to 4, wherein in general formulas (2) and (3), the substituents represented by R₂₇ and R₃₅ are each an alkyl group with 1 to 10 carbon atoms, an aryl group with 6 to 12 carbon atoms, or a heteroaryl group with 3 to 12 carbon atoms.

### (Constitution 6)

The organic compound of any one of Constitutions 2 to 5, wherein in general formulas (2) and (3), X₁ to X₄ are each an oxygen atom or a sulfur atom.

### (Constitution 7)

The organic compound of any one of Constitutions 2 to 6, wherein general formulas (2) and (3) have no structure represented by general formula (4):

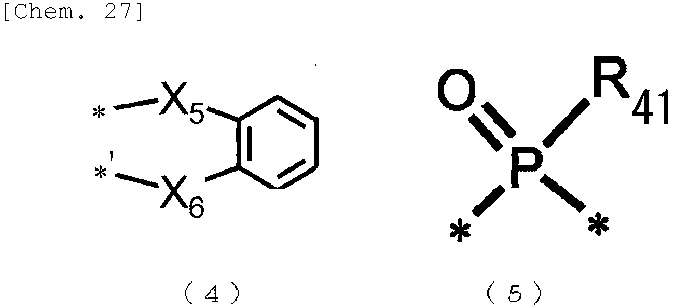

In general formula (4), X₅ and X₆ are each independently selected from an oxygen atom, a sulfur atom, a dimethylmethylene group, a sulfonyl group, and the structure represented by general formula (5), and asterisk * indicates the position of a bond to R₂₆ or R₃₄, and *' indicates the position of a bond to R₂₇ or R₃₅.

In general formula (5), R₄₁ represents a phenyl group, and the asterisks * indicate binding positions.

### (Constitution 8)

An organic light-emitting element including: a first electrode and a second electrode; and an organic compound layer disposed between the first electrode and the second electrode, wherein the organic compound layer contains the organic compound of any one of Constitutions 1 to 7.

### (Constitution 9)

The organic light-emitting element of Constitution 8, wherein the organic compound layer includes a luminescent layer, and the luminescent layer contains the organic compound.

### (Constitution 10)

The organic light-emitting element of Constitution 9, wherein the luminescent layer further contains a first compound, and the first compound has a higher lowest excited singlet energy than the organic compound.

### (Constitution 11)

The organic light-emitting element of Constitution 10, wherein the first compound has an aryl group with 6 to 18 carbon atoms.

### (Constitution 12)

The organic light-emitting element of Constitution 10 or 11, wherein the first compound is a hydrocarbon compound.

### (Constitution 13)

The organic compound of any one of Constitutions 10 to 12, wherein the first compound has at least one of a pyrene skeleton, a triphenylene skeleton, and a chrysene skeleton.

### (Constitution 14)

The organic light-emitting element of any one of Constitutions 10 to 13, the luminescent layer further contains a third compound, and the third compound has a lower lowest excited singlet energy than the organic compound.

### (Constitution 15)

The organic light-emitting element of Constitution 14, wherein the third compound has a lower lowest excited triplet energy than the organic compound.

### (Constitution 16)

The organic light-emitting element of Constitution 14 or 15, wherein the first compound has a higher lowest excited singlet energy than the third compound.

### (Constitution 17)

A display device including: a plurality of pixels, at least one of the pixels including the organic light-emitting element as set forth in any one of Constitutions 8 to 16; and a transistor connected to the organic light-emitting element.

### (Constitution 18)

A photoelectric conversion device including: an optical section including a lens; an imaging element operable to receive light that has passed through the optical section; and a display section on which an image taken by the imaging element is displayed, wherein the display section includes the organic light-emitting element as set forth in any one of Constitutions 8 to 16.

### (Constitution 19)

An electronic apparatus including: a display section including the organic light-emitting element as set forth in any one of Constitutions 8 to 16; a housing provided with the display section; and a communication section provided at the housing, the communication section being operable to communicate with the outside.

### (Constitution 20)

A lighting device including: a light source including the organic light-emitting element as set forth in any one of Constitutions 8 to 16; and a light diffusing section or an optical film that transmits light emitted from the light source.

### (Constitution 21)

A movable apparatus including: a lighting fixture including the organic light-emitting element as set forth in any one of Constitutions 8 to 16; and an apparatus body provided with the lighting fixture.

### (Constitution 22)

An image forming apparatus including: a photosensitive member; and an exposure light source operable to expose the photosensitive member to light, wherein the exposure light source includes the organic light-emitting element as set forth in any one of Constitutions 8 to 16.

### (Constitution 23)

A wearable device including: a display section including the organic light-emitting element as set forth in any one of Constitutions 8 to 16; an optical system operable to collect light from the display section; and a controller operable to control the displaying operation of the display.

The present invention is not limited to the above-described embodiments, and various modifications and changes may be made without departing from the spirit and scope of the invention. Accordingly, the claims will be appended below to make public the scope of the invention.

This application claims the priority based on Japanese Patent Application No. 2022-018104 filed February 8, 2022 and No. 2022-199794 filed December 14, 2022, which are hereby incorporated by reference herein in their entirety.

### Reference Signs List

- 1: interlayer insulating layer
- 2: reflection electrode
- 3: insulating layer
- 4: organic compound layer
- 5: transparent electrode
- 6: protective layer
- 7: color filter
- 10: sub-pixel
- 11: substrate
- 12: insulating layer
- 13: gate electrode
- 14: gate insulating film
- 15: semiconductor layer
- 16: drain electrode
- 17: source electrode
- 18: thin film transistor
- 19: insulating film
- 20: contact hole
- 21: lower electrode
- 22: organic compound layer
- 23: upper electrode
- 24: first protective layer
- 25: second protective layer
- 26: organic light-emitting element
- 100: display device
- 1000: display device
- 1001: upper cover
- 1002: flexible printed circuit
- 1003: touch panel
- 1004: flexible printed circuit
- 1005: display panel
- 1006: frame
- 1007: circuit board
- 1008: battery
- 1009: lower cover
- 1100: imaging device
- 1101: viewfinder
- 1102: rear display
- 1103: operational section
- 1104: housing
- 1200: electronic apparatus
- 1201: display section
- 1202: operational section
- 1203: housing
- 1300: display device
- 1301: housing
- 1302: display section
- 1303: base
- 1310: display device
- 1311: first display section
- 1312: second display section
- 1313: housing
- 1314: folding point
- 1400: lighting device
- 1401: housing
- 1402:: light source
- 1403: circuit board
- 1404: optical film
- 1405: light diffusing section
- 1500: automobile
- 1501: tail lamp
- 1502: window
- 1503: car body
- 1600: smart glasses
- 1601: lens
- 1602: imaging device
- 1603: controller
- 1610: smart glasses
- 1611: lens
- 1612: controller

## Claims

1. An organic compound represented by general formula (1): 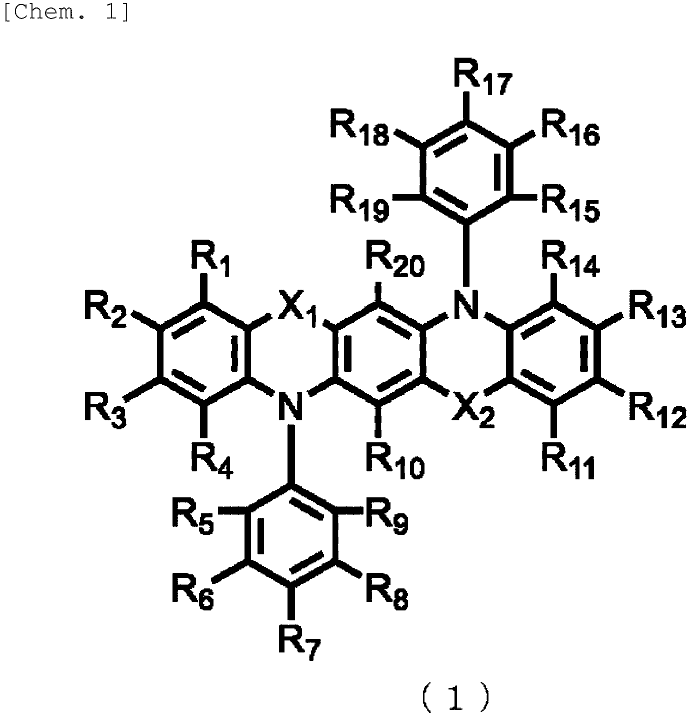 (wherein in general formula (1), R₁ to R₂₀ are each independently selected from the group consisting of a hydrogen atom, a deuterium atom, halogen atoms, substituted or unsubstituted alkyl groups, substituted or unsubstituted alkoxy groups, substituted or unsubstituted silyl groups, substituted or unsubstituted amino groups, substituted or unsubstituted aryl groups, substituted or unsubstituted heteroaryl groups, and a cyano group, wherein R₁ to R₂₀ may be bound to one another to form a ring and, optionally, via a chalcogen atom, wherein at least a combination R₉ and R₁₀ or R₁₉ and R₂₀ forms a bond, and wherein X₁ and X₂ are each independently selected from the group consisting of chalcogen atoms, substituted or unsubstituted imino groups, substituted or unsubstituted methylene groups, and substituted or unsubstituted silylene groups.)

2. The organic compound according to Claim 1, wherein the organic compound is represented by general formula (2) or (3) : 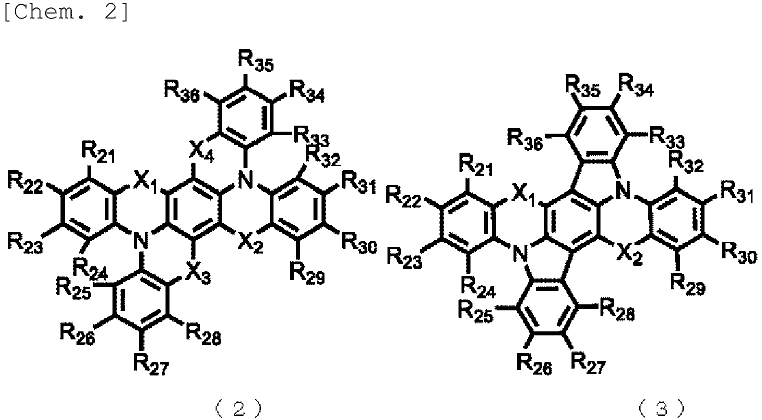 (wherein in general formulas (2) and (3), R₂₁ to R₃₆ are each independently selected from the group consisting of a hydrogen atom, a deuterium atom, halogen atoms, substituted or unsubstituted alkyl groups, substituted or unsubstituted alkoxy groups, substituted or unsubstituted silyl groups, substituted or unsubstituted amino groups, substituted or unsubstituted aryl groups, substituted or unsubstituted heteroaryl groups, and a cyano group, wherein R₂₁ to R₃₆ may be bound to one another to form a ring and, optionally, via a chalcogen atom, and wherein X₁ to X₄ are each independently selected from the group consisting of chalcogen atoms, substituted or unsubstituted imino groups, substituted or unsubstituted methylene groups, and substituted or unsubstituted silylene groups.)

3. The organic compound according to Claim 2, wherein in general formulas (2) and (3), at least one of R₂₁, R₂₂, R₂₃, R₂₆, R₂₇, R₂₉, R₃₀, R₃₁, R₃₄, and R₃₅ is other than the hydrogen atom.

4. The organic compound according to Claim 2, wherein in general formulas (2) and (3), at least one of R₂₁, R₂₂, R₂₃, R₂₆, R₂₇, R₂₉, R₃₀, R₃₁, R₃₄, and R₃₅ is an alkyl group with 1 to 10 carbon atoms, an aryl group with 6 to 12 carbon atoms, or a heteroaryl group with 3 to 12 carbon atoms.

5. The organic compound according to Claim 2, wherein in general formulas (2) and (3), the substituents represented by R₂₇ and R₃₅ are each an alkyl group with 1 to 10 carbon atoms, an aryl group with 6 to 12 carbon atoms, or a heteroaryl group with 3 to 12 carbon atoms.

6. The organic compound according to Claim 2, wherein in general formulas (2) and (3), X₁ to X₄ are each an oxygen atom or a sulfur atom.

7. The organic compound according to Claim 2, wherein general formulas (2) and (3) have no structure represented by general formula (4): 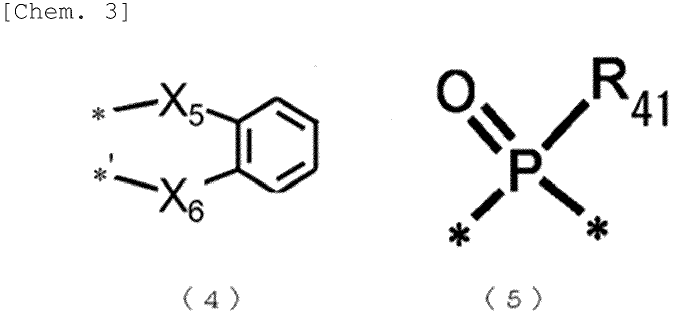
wherein in general formula (4), X₅ and X₆ are each independently selected from an oxygen atom, a sulfur atom, a dimethylmethylene group, a sulfonyl group, and a structure represented by general formula (5), and asterisk * indicates the position of a bond to R₂₆ or R₃₄, and *' indicates the position of a bond to R₂₇ or R₃₅, and
wherein in general formula (5), R₄₁ represents a phenyl group, and asterisks * indicate binding positions.

8. An organic light-emitting element comprising:
a first electrode and a second electrode; and
an organic compound layer disposed between the first electrode and the second electrode,
wherein the organic compound layer contains the organic compound as set forth in Claim 1.

9. The organic light-emitting element according to Claim 8, wherein
the organic compound layer includes a luminescent layer, and
the luminescent layer contains the organic compound.

10. The organic light-emitting element according to Claim 9, wherein
the luminescent layer further contains a first compound, and
the first compound has a higher lowest excited singlet energy than the organic compound.

11. The organic light-emitting element according to Claim 10, wherein the first compound is a hydrocarbon compound.

12. The organic compound according to Claim 10, wherein the first compound has at least one of a pyrene skeleton, a triphenylene skeleton, and a chrysene skeleton.

13. The organic light-emitting element according to Claim 10, wherein
the luminescent layer further contains a third compound, and
the third compound has a lower lowest excited singlet energy than the organic compound.

14. The organic light-emitting element according to Claim 13, wherein the first compound has a higher lowest excited singlet energy than the third compound.

15. A display device comprising: a plurality of pixels, at least one of the pixels including the organic light-emitting element as set forth in any one of Claims 8 to 14; and a transistor connected to the organic light-emitting element.

16. A photoelectric conversion device comprising: an imaging element operable to receive light; and a display section on which an image taken by the imaging element is displayed,
wherein the display section includes the organic light-emitting element as set forth in any one of Claims 8 to 14.

17. An electronic apparatus comprising: a display section including the organic light-emitting element as set forth in any one of Claims 8 to 14; a housing provided with the display section; and a communication section provided at the housing, the communication section being operable to communicate with the outside.

18. A lighting device comprising: a light source including the organic light-emitting element as set forth in any one of Claims 8 to 14; and a light diffusing section or an optical film that transmits light emitted from the light source.

19. A movable apparatus comprising: a lighting fixture including the organic light-emitting element as set forth in any one of Claims 8 to 14; and an apparatus body provided with the lighting fixture.

20. An image forming apparatus comprising: a photosensitive member; and an exposure light source operable to expose the photosensitive member to light,
wherein the exposure light source includes the organic light-emitting element as set forth in any one of Claims 8 to 14.

21. A wearable device comprising: a display section including the organic light-emitting element as set forth in any one of Claims 8 to 14; an optical system operable to collect light from the display section; and a controller operable to control the displaying operation of the display.
